# EUROPEAN PATENT APPLICATION

(11) **EP 2 816 108 A1**
(43) Date of publication of application: **24.12.2014**
(21) Application number: 13173185.3
(22) Date of filing: 21.06.2013
(51) Int. Cl.: C12N 9/04, C12N 9/88, C07K 14/745, C12N 1/14, C12P 21/02, C12R 1/645

(54) **Transformed fungus with vitamin K dependent activity**

(71) Applicant: Baxter International Inc, Deerfield, IL 60015 (US); Baxter Healthcare SA, 8152 Glattpark (Opfikon) (CH)
(72) Inventor: Scheiflinger, Friedrich, 1090 Vienna (AT); Dattenböck, Christoph, 1080 Vienna (AT); Strauss, Joseph, 1120 Vienna (AT)
(74) Representative: Sonn & Partner Patentanwälte

(57) **Abstract**

The present invention provides a fungus cell transfected with exogenous nucleotide coding sequence of a gamma-carboxylase, a vitamin K reductase, and a vitamin K-dependent polypeptide, wherein the gamma-carboxylase and vitamin K reductase sequences lack an ER retention sequence endogenous to said fungus cell, preferably are free of any fungal ER retention leader sequence.

## Description

The present invention relates to the field of recombinant protein expression in enzyme transfected fungal cells.

Recombinant human blood clotting factors are valuable pharmaceuticals in the treatment of haemophilia. The production of these therapeutic proteins is currently achieved in mammalian cell cultures. Fungi are promising alternatives, because they provide a well-studied economic system, in which cells can be grown in chemically defined media in the absence of animal derived material. However, for the production of functional vitamin K dependent (VKD) clotting factors, fungal cells have to be modified, owing to their inability to oxidize glutamate (glu) residues of VKD proteins in a posttranslational (PT) reaction. The reaction is performed presumably in the endoplasmic reticulum by VKD-carboxylase (GGCX), whereby specific glu residues in VKD proteins are modified to carboxy glutamic acid (gla). In this process reduced vitamin K is converted to vitamin K epoxide, which is subsequently recycled to vitamin K by the ER-membrane resident vitamin K epoxide reductase (VKOR).

The most common form of blood clotting disorder is hemophilia A (factor VIII deficiency) followed by hemophilia B (factor IX deficiency). Till now there is no cure for hemophilia, though the disease can be treated by replacement therapy with the missing functional clotting factor (Pipe, 2010). Before the age of recombinant protein technology, clotting factors were obtained from pooled plasma fractions. In the 1980s it was observed that HIV and hepatitis C were transmitted through plasma-derived factor VIII to hemophilia patients (Grillberger et al., 2009). The catastrophic consequences for the infected hemophilia patients were one of the driving forces for the development of virus-free, recombinant clotting factors (Pipe, 2008). Since then genes encoding factors VIII, IX and VIIa were cloned and expressed in mammalian cell culture. However, recombinant clotting factor production was challenging due to the complexity and extensive post-translational modifications. In contrast to factor VIII, factor IX and VIIa receive a gamma-carboxylation of glutamic acid residues within the so-called 'gla domain' next to the N-terminus. Gamma-carboxylation of glutamate residues is essential to bind calcium ions, enabling the proper folding of the gla domain. The folded gla domain interacts with negatively charged phospholipid surfaces, and this is essential for biological activity (Berkner and Runge, 2004; Hansson and Stenflo, 2005). Production of gamma-carboxylated factor IX was reported in chinese hamster ovary (CHO), baby hamster kidney (BHK) as well as human embryonic kidney-293 cells (Pipe, 2010). Commercial large scale fermentation for factor IX was demonstrated to be mostly efficient in CHO cells. Overexpression of factor IX led to an immense amount of secreted inactive protein. It was recognized that the carboxylation machinery as well as the proteolytic processing of the propeptide was already saturated. The problem with unprocessed propeptides could be bypassed by co-expression of furin. In contrast, the overexpression of gamma-carboxylase resulted in a decreased factor IX secretion, although an increment in factor IX/gamma-carboxylase complexes was observed (Hallgren et al., 2002). Hallgren et al. (2002) suggested that the vitamin K cofactor was the rate limiting step in carboxylation. In another study, the role of VKORC1 was confirmed to be the rate-limiting step in the gamma-carboxylation system (Wajih et al., 2005).

Apart from mammalian cell culture systems, pilot production of gamma-carboxylated recombinant factor IX was reported from transgenic sow milk (Sun et al., 2012). Moreover, Vatandoost and coworkers reported the expression of factor IX in Drosophila S2 cells (Vatandoost et al., 2012). However, Drosophila melano-gaster carries both genes necessary for gamma-carboxylation and vitamin K epoxide recycling. The co-expression of VKORC1 and GGCX in Sf21 *Spodoptera* insect cells, which do not contain endogenous gamma-carboxylase, VKORC1 or vitamin K dependent pro-tein, was shown by (Rishavy et al., 2011). However, the complete engineering of GGCX, VKORC1 and a VKD reporter protein has not reported so far in insect cells. Apart from mammalian and insect cell systems, Oldenburg et al. (2006) reported that activity of VKORC1 can be assayed when expressed in the yeast *Pichia pastoris.* In another study it was observed that, the bimodular oxi-doreductase of *A. thaliana* (At4g35760) comprises a domain which is homologous to the catalytic subunit of mammalian VKORC1 (Furt et al., 2010). The VKORC1 like domain with an added C-terminal ER-retention signal was expressed in yeast and shown to be active.

WO 2006/067116 A1 describes methods for preparing vitamin K-dependent polypeptides in a carboxylation-deficient host cells, comprising co-expression of a gamma-carboxylase, a vitamin K reductase, such as vitamin K 2,3-epoxide reductase (VKOR) and a Vitamin K-dependent polypeptide. Host cells are particularly CHO (hamster), sf9 (insect) and yeast cells. For CHO and insect cell expression of gamma-carboxylase and VKOR, the entire human gene, including human promoters and signal sequences was used. For expression in yeast, a MF(alpha)1 signal sequence was ligated to the encoding sequence. Also mentioned are other fungi, such as *A. nidulans.* However information on protein expression, stability and activities are generic and no particular instructions are given for any of these cells.

Fungi occupy a large spectrum of habitats, including soil, decaying organic materials, plant cell material or indoor environments (Fleissner and Dersch, 2010). Fungi secrete a broad range and high levels of enzymes, which enable them to utilize various organic compounds as nutrients. Fungi became attractive as effective production hosts for extracellular (homologous) enzymes (Meyer et al., 2011). Beyond several classes of technical enzymes, thermostable glucoamylase is one of the best studied industrial enzymes. Glucoamylase is mainly produced by *Aspergillus* species and typical fermentation yields of the enzyme are in the range of 25 g/L (Ward et al., 2006). Major improvements in developing filamentous fungi to biotechnological workhorses were achieved in the 1980s, when Kinghorn and Hawkins showed first reports of gene cloning and Tilburn and coworkers succeeded in transformation of *Aspergillus* (Tilburn et al., 1983; Kinghorn and Hawkins, 1982). Since then filamentous fungi were developed to a heterologous protein production platform, which offers an alternative system to mammalian systems or *E.coli.* Filamentous fungi have a more rigid cell wall compared to mammalian or insect cells, which makes them more resistant to shear stress and allows them to grow in high cell densities, which facilitates industrial scale up. On the other hand, the cell wall can hamper recombinant protein secretion (Meyer et al., 2011, Kinghorn and Unkles, 1993; Fleissner and Dersch, 2010; Lubertozzi and Keasling, 2009). In the phylogenetic kingdom of fungi no VKOR or GGCX homologues have been found.

In preliminary experiments to the invention it has been found that creating a fungus system for vitamin K-dependent polypeptides by supplementing gamma-carboxylase and vitamin K reductase to a fungus is not trivial and failed to obtain any significant product yields if general instructions of WO 2006/067116 A1 are followed.

It is therefore a goal of the invention to provide methods that allow the expression of vitamin K-dependent polypeptides, such as prothrombin.

According to a first aspect, the present invention provides a fungus cell stably or transiently transformed with exogenous nucleotide coding sequence of a gamma-carboxylase, a vitamin K reductase, and a vitamin K-dependent polypeptide, wherein the gamma-carboxylase and vitamin K reductase sequences lack an ER retention sequence endogenous to said fungal cell, preferably are free of any fungal ER retention sequence.

A gamma-carboxylase (also referred to as "gamma-glutamyl carboxylase" or "GGCX") is an enzyme that gamma-carboxylates glutamic acid residues on target proteins, usually said reaction requires vitamin K hydroquinone as cofactor.

A vitamin K reductase (also referred to as "vitamin K epoxide reductase" or "VKOR") reduces vitamin K epoxide to vitamin K quinone and then to vitamin K hydroquinone. It has been shown that warfarin-insensitive NAD(P)H dehydrogenase (NQO1) reduces vitamin K and can support VKD gamma-carboxylation (Wallin and Hutson 1982). However, Tie and coworker showed evidence of a warfarin-sensitive enzyme, which reduces vitamin K to vitamin K hydroquinone and is probably not NQO1 (Tie et al. 2011). Vitamin K hydroquinone is in turn oxidised during carboxylation of glutamate ("Glu") to gamma-carboxyglutamate ("Gla") by the gamma-glutamyl carboxylase. Glutamate and gamma-carboxyglutamate may be in the protonated (acidic) or deprotonated form; "Glu" and "Gla" refers to these forms equally.

The term "vitamin K-dependent polypeptide" (also referred to as "VKD"), as used herein, represents any amino acid sequence, which can be posttranslationally modified by an enzyme using vitamin K hydroquinone as cofactor and is gamma-carboxylated on glutamic acid residues. In particular said enzyme is a gamma-glutamyl carboxylase. Accordingly, the vitamin K-dependent polypeptide can bind a gamma-glutamyl carboxylase. Typical vitamin K-dependent polypeptides comprise one or more binding site or recognition sequences for interaction with gamma-glutamyl carboxylase, which carboxylates glutamate residues usually located in the Gla domain of vitamin K-dependent proteins.

Gamma-carboxylation is a process that has been shown to occur and presumably always occurs in the ER (endoplasmatic reticulum). The involved enzymes were required to contain ER retention leader signals for proper localisation in the ER to achieve catalytic activity. The use of ER retention leader signals, such as the leader sequence of *Aspergillus niger* MnsA - the fungus equivalent of the yeast Mns1 signal sequence, resulted in poor and unstable expression of GGCX and VKOR. Surprisingly, no enzyme activities were detected, which seemed to be caused by low protein stability. The presence of ER retention sequences endogenous to said fungal cell, e.g. the *Aspergillus niger* MnsA ER retention sequence in the expression in *Aspergillus,* had a detrimental effect on protein stability and expression yield. Other non-endogenous ER retention sequences may or may not be present according to the invention. Surprisingly, native codon optimized GGCX and VKORC1 sequences led to activity and proper gamma-carboxylation of VKDs. Thus in a second aspect of the present invention, a fungal cell is provided comprising a codon optimized gamma-carboxylase and vitamin K reductase coding sequence, such as a gamma-carboxylase provided by SEQ ID NO: 1 or a sequence being at least 70%, preferably at least 75%, or at least 80%, at least 85%, at least 90%, at least 95%, at least 98%, identical with the entire SEQ ID NO:1 ; or a vitamin K reductase provided by SEQ ID NO: 4 or a sequence being at least 70%, preferably at least 75%, or at least 80%, at least 85%, at least 90%, at least 95%, at least 98%, identical with the entire SEQ ID NO: 4. This second aspect can be combined with any features or preferred embodiments of the first aspect of the present invention. "Codon optimized" means that a nucleic acid sequence is used, wherein codons of a transgene are replaced by codons abundant in the fungal cell of interest, especially *Aspergillus.* The fungal cell may further comprise a nucleic acid sequence coding far any VDK. Abundant codons for Aspergillus nidulans (also called Emericella nidulans(Teleomorph)) are provided in table 1.

**Table 1: Aspergillus nidulans (Emericella nidulans) codon frequencey (746 CDS's, 443998 codons), source: www.kazusa.or.jp/codon/cgi-bin/showcodon.cgi?species=162425 fields: [triplet] [frequency: per thousand] ([number])**

| | | | |
|---|---|---|---|
| UUU 13.5 ( 5998) | UCU 15.2 ( 6737) | UAU 11.6 ( 5156) | UGU 4.5 ( 2008) |
| UUC 25.2 ( 11196) | UCC 16.8 ( 7464) | UAC 19.3 ( 8558) | UGC 7.8 ( 3444) |
| UUA 5.0 ( 2240) | UCA 11.1 ( 4912) | UAA 0.6 ( 267) | UGA 0.6 ( 260) |
| UUG 13.5 ( 5974) | UCG 13.8 ( 6146) | UAG 0.5 ( 241) | UGG 13.4 ( 5957) |
| | | | |
| CUU 18.9 ( 8381) | CCU 15.9 ( 7042) | CAU 10.6 ( 4726) | CGU 10.6 ( 4703) |
| CUC 23.3 ( 10331) | CCC 15.3 ( 6814) | CAC 12.9 ( 5726) | CGC 16.0 ( 7086) |
| CUA 8.1 ( 3582) | CCA 11.9 ( 5284) | CAA 15.3 ( 6813) | CGA 9.4 ( 4159) |
| CUG 20.0 ( 8863) | CCG 13.9 ( 6151) | CAG 25.5 ( 11343) | CGG 9.5 ( 4236) |
| | | | |
| AUU 18.8 ( 8335) | ACU 14.4 ( 6379) | AAU 15.2 ( 6762) | AGU 9.1 ( 4044) |
| AUC 26.8 ( 11905) | ACC 19.8 ( 8805) | AAC 25.5 ( 11305) | AGC 15.9 ( 7059) |
| AUA 5.6 ( 2508) | ACA 13.0 ( 5785) | AAA 15.8 ( 7018) | AGA 5.9 ( 2619) |
| AUG 21.9 ( 9712) | ACG 12.2 ( 5435) | AAG 31.9 ( 14168) | AGG 6.0 ( 2672) |
| | | | |
| GUU 17.7 ( 7853) | GCU 23.4 ( 10400) | GAU 26.3 ( 11663) | GGU 19.5 ( 8663) |
| GUC 24.1 ( 10709) | GCC 26.6 ( 11818) | GAC 29.9 ( 13294) | GGC 24.9 ( 11066) |
| GUA 5.9 ( 2614) | GCA 16.3 ( 7216) | GAA 25.0 ( 11086) | GGA 14.7 ( 6545) |
| GUG 14.6 ( 6474) | GCG 17.3 ( 7682) | GAG 35.8 ( 15897) | GGG 10.6 ( 4709) |

Coding GC 53.19% 1st letter GC 56.97% 2nd letter GC 43.54% 3rd letter GC 59.06%

The codon usage table can be calculated from sequences of CDS (complete coding sequences). The codon usage table differentiates slightly between different *Aspergillus* species. In general, an improvement in protein expression can be expected by the use of genes with a codon bias, which is closely related to that of the fungal host (Gouka 1997, MacKenzie 2004).

In particular, the invention demonstrated that VKD, such as prothrombin, with its native sequence can be successfully gamma-carboxylated and secreted into the fungal culture supernatant. Also, gamma-carboxylated VKD proteins can be harvested and purified from the cells, e.g. from total cell lysates.

Thus, according to the invention the GGCX lacks an endogenous and/or exogenous ER retention leader sequence. Further, the VKOR lacks an endogenous and/or exogenous ER retention leader sequence. "Endogenous" and "exogenous" as used herein relates to the fungus cell used for the inventive recombinant expression of a VDK. Within the meaning of "endogenous", the GGCX and/or VKOR may lack any fungal ER retention leader sequence, or lack an ER retention leader sequence of the particular fungus species or fungus genus or fungus family. "Exogenous" relates to non-fungus sequences.

The GGCX, VKOR and/or VKD sequences are transgenes for the fungus, i.e. the fungal host is gamma-carboxylation-deficient and lacks the 'vitamin K cylce'. Preferably GGCX, VKOR and/or VKD are vertebrate sequences, preferably of a mammal, e.g. they may be sequences of human, mouse, rat, hamster or primate proteins.

In further preferred embodiments, the fungal cell is an *Aspergillus* cell, preferably an *Aspergillus nidulans* cell. The fungus may be any filamentous or yeast-like fungus. "Fungus cell" as used herein may refer to an isolated cell, i.e. a cell not attached to other cells, or a cell in a cellular aggregate, in particular a fungus or fungus filament. Further examples of fungi are *Neurospora* spp., *Fusarium* spp. or *Trichoderma* spp.. Further *aspergillus* species are *A. oryzae, A. nidulans* or *A. ni*ger. Yeast-type fungal cells are e.g. *Saccharomyces, Schizosac-charomyces* or *Pichia* cells, in particular a *Pichia pastoris, Saccharomyces cerevisiae,* or *Schizosaccharomyces pombe* cell.

The gamma-carboxylase protein is preferably as set forth in SEQ ID NO: 3 (human gamma-carboxylase, UniProtID P38435). Any coding nucleotide sequence can be used. Preferably the nucleotide sequence for gamma-carboxylase is codon optimized, in particular preferred the coding sequence of the gamma-carboxylase comprises a sequence selected from SEQ ID NO: 1 or a sequence being at least 70%, preferably at least 75%, or at least 80%, at least 85%, at least 90%, at least 95%, at least 98%, identical with the entire SEQ ID NO: 1. Such sequences achieve improved expression and consequently higher activity of the cell. The human wild type gamma-carboxylase sequence (NCBI database acc. NM_000506) is provided in SEQ ID NO: 2 and can also be used in certain less preferred embodiments. The codon optimized sequence has a 78.1% sequence identity (1783/2284) to the wild type sequence.

Codon optimization can be performed by replacing codons coding for a particular amino acid by another codon coding for the same amino acid. The new codon should facilitate a smooth translation and/or transcription in the cell. Preferably codons which are abundant in the particular cell are used, as can be determined by statistical analysis of the known coding sequences of the host cell organism.

The vitamin K reductase protein is preferably as set forth in SEQ ID NO: 6 (human vitamin K reductase, UniProtID Q9BQB6). Any coding nucleotide sequence can be used. Preferably the nucleotide sequence for vitamin K reductase is codon optimized, in particular preferred the coding sequence of the vitamin K reductase comprises a sequence selected from SEQ ID NO: 4 or a sequence being at least 70%, preferably at least 75%, or at least 80%, at least 85%, at least 90%, at least 95%, at least 98%, identical with the entire SEQ ID NO: 4, preferably a sequence with the V29L mutation or V29M mutation. Such sequences have a sufficient expression rate. The human wild type vitamin K reductase sequence (NCBI database acc. NM_024006) is provided in SEQ ID NO: 5 and can also be used in certain less preferred embodiments. The codon optimized sequence has a 80.2% sequence identity (392/489) to the wild type sequence. The V29M mutation might be used to confirm ER localization in the absence or presence of an endogenous/exogenous ER retention leader sequence.

The vitamin K-dependent polypeptide can be selected from prothrombin, factor VII, factor IX, factor X, protein C, protein S, protein Z, pulmonary surfactant-associated proteins, osteocalcin, proline-rich GIa protein 1, and matrix gla-protein. Especially preferred, the vitamin K-dependent polypeptide is prothrombin.

The prothrombin protein is preferably as set forth in SEQ ID NO: 9 (human prothrombin, UniProtID P00734). Any coding nucleotide sequence can be used. Preferably the nucleotide sequence for prothrombin is codon optimized, in particular preferred the coding sequence comprises a sequence selected from SEQ ID NO: 7 or a sequence being at least 70%, preferably at least 75%, or at least 80%, at least 85%, at least 90%, at least 95%, at least 98%, identical with the entire SEQ ID NO: 7. Such sequences achieve improved expression and consequently higher yields. The human wild type prothrombin sequence (NCBI database acc. NM_000506) is provided in SEQ ID NO: 8 and can also be used in certain less preferred embodiments. The codon optimized sequence has a 81.7% sequence identity (1528/1870) to the wild type sequence.

As used herein, sequence identities relate to identical nucleotides of a complete template sequence found in a query sequence after a suitable sequence alignment, such as by the EMBOSS Needle program using default parameters. Template sequences are e.g. the sequences provided in the sequence listing as SEQ ID NOs 1-9.

Advantageously, the gamma-carboxylase and the vitamin K reductase are expressed in substantially the same amounts or by increased vitamin K reductase expression as compared to gamma-carboxylase expression (molar ratio), e.g. by adjusting the gene copy number or expression intensity by selecting suitable promoters. Substantially the same amounts means the ratio of gamma-carboxylase to vitamin K reductase expression is in the range of 1:20 to 20:1, preferably 1:10 to 15:1, even more preferred 1:5 to 10:1, or 1:2 to 5:1, in preferred embodiments 1:2 to 2:1. Especially preferred, the expression intensities of vitamin K reductase is higher compared to gamma-carboxylase expression intensity for efficient gamma carboxylation. Preferably the genes of said gamma-carboxylase and vitamin K reductase comprise the same promoter or a different promoter. The promoters can be inducible promoters, such as an estrogen inducible promoter. Different inducible promoters provide an easy way to adjust the expression rate ratios mentioned above and are used according to a preferred embodiment of the invention. Example promotes are the alcA promoter and the EREuraRS promoter, e.g. the alcA promoter is used for the vitamin K reductase and the EREuraRS promoter for the gamma-carboxylase.

The nucleic acid is preferably in the host cell chromosome to obtain a recombinant host cell. This integration is generally considered to be an advantage as the DNA sequence is more likely to be stably maintained in the cell. Integration of the DNA constructs into the host chromosome may be performed according to conventional methods, e.g. by homologous or heterologous recombination. In particular preferred, said gamma-carboxylase and vitamin K reductase have been introduced into the genome of said cell in an amount ratio of gamma-carboxylase to vitamin K reductase integrations of 1:5 to 5:1. This allows similar expression amounts and an adequate performance of the vitamin K cycle. The ratio can be obtained by transforming a multitude of cells individually and selecting a cell with said ratio. Such rations can be tested by quantitative PCR methods using the genome of the cell. Usually multiple copies of the gamma-carboxylase and the vitamin K reductase are introduced into the fungus cell, especially into the genome of the fungus cell.

In a further aspect, the invention provides a method of gamma-carboxylating a vitamin K-dependent polypeptide in a cell according to the invention, comprising expressing the gamma-carboxylase, vitamin K reductase, and vitamin K-dependent polypeptide in the presence of vitamin K or a vitamin K precursor, such as vitamin K hydroquinone, in said cell.

The product, the gamma-carboxylated vitamin K-dependent polypeptide is usually secreted to the cell or fungus culture medium or supernatant, where it can be harvested. Also, the gamma-carboxylated vitamin K-dependent polypeptide can be obtained from intracellular contents by separation from other cell components and purification. E.g., gamma-carboxylated vitamin K-dependent polypeptide can be purified from total cell extracts.

To allow optimal supply of the exogenous cofactor vitamin K (or its hydroquinone) it is preferably administered to the culture. Especially advantageous was the administration of vitamin K or said precursor to said cell wherein said vitamin K or precursor is provided in a micelle. The provision in a micelle surprisingly led to better distribution and provision of the cell and resulted in higher product yields.

Preferably the cell or fungus is grown in a suitable medium or culture condition. Fungi usually require little external nutrients as a carbon source. The carbon source is preferably in sufficient amount to prevent a vitamin K digestion, which is usually also provided in the medium. A growth medium may further optionally comprise a nitrogen source, amino acids, sugars, vitamins, salts, phospholipids, protein and/or growth factors. Preferably salts include one or more cations selected from Na, K, Zn, Fe, Mg, Mn, Co, Cu, Mo, NH₄ and/or one or more anions selected from SO₄ BO₃, Cl, PO₄. A preferred nitrogen source is an ammonium ion, e.g. in form of ammonium tartrate. Preferably the pH of the medium is between 5.5 and 8.5, especially preferred between 6.5 and 8. The carbon source preferably comprises a carbohydrate, especially a hexose or pentose or any polymers thereof, preferably a mono-hexose or mono-pentose. Preferably the carbon source is in a concentration of at least 0.01 % (w/v), at least 0.05 % (w/v), at least 0.1 % (w/v) or at least 0.15% (w/v), e.g. in the range of 0.01% to 10 % (w/v) or 0.05% to 5% or even 0.1% to 1% (w/v). The carbon source can be any carbon source utilized by the fungal cell, but preferably glucose or fructose.

Especially preferred, the medium according to the present invention comprises vitamin K, especially vitamin K1. It may also comprise DES (Diethylstilbestrol) and/or EMK (ethylmethylketone).

In preferred embodiments the cell expresses polypeptides according to SEQ ID NO: 3 (human gamma-carboxylase) and/or SEQ ID NO: 6 (human vitamin K reductase), preferably further a polypeptide of SEQ ID NO: 9 (human prothrombin), or a sequence being at least 70%, preferably at least 75%, or at least 80%, at least 85%, at least 90%, at least 95%, at least 98%, identical to any one of SEQ ID NO: 3, 6 or 9.

The present invention further relates to a method of manufacturing a fungus cell comprising the step of providing a fungus cell and introducing into said cell an exogenous nucleotide coding sequence of a gamma-carboxylase and an exogenous nucleotide coding sequence a vitamin K reductase. Alternatively it is possible to provide a fungus cell transfected with an exogenous nucleotide coding sequence of a gamma-carboxylase and a further fungus cell transfected with an exogenous nucleotide coding sequence a vitamin K reductase said method further comprises the step of crossing said cells thereby obtaining a descendant cell comprising an exogenous nucleotide coding sequence of a gamma-carboxylase and an exogenous nucleotide coding sequence a vitamin K reductase. Said transfections should be stable, in particular into the genome of said cells.

The method may further comprise the step of transfecting one of said cell transfected with a coding sequence for said vitamin K-dependent polypeptide or crossing one of said cells with a cell comprising a nucleic acid sequence of the vitamin K-dependent polypeptide resulting in a descendant cell comprising said gamma-carboxylase, vitamin K reductase, and vitamin K-dependent polypeptide coding sequences.

In preferred embodiments of the inventive methods, the cells are cultured in the presence of carbonate and/or phosphate, especially preferred carbonate. In particular, the treatment with calcium and/or phosphate can be a pre-treatment before transfecting the cells with the inventive nucleic acid molecules or a pre-treatment before expression, e.g. before induction of expression. In addition or alternatively, it is also possible to treat the cells with carbonate and/or phosphate during the transfection and/or during the expression.

The inventive cell is further used to produce the vitamin K-dependent polypeptide.

Vitamin K-dependent proteins of the present invention can be used to treat certain types of hemophilia. Hemophilia A is characterized by the absence of active factor VIII, factor VIIIa, or the presence of inhibitors to factor VIII. Hemophilia B is characterized by the absence of active factor IX, factor IXa. FVII deficiency, although rare, responds well to factor VII administration. Factor VIII replacement therapy is limited due to development of high-titer inhibitory factor VIII antibodies in some patients. Alternatively, FVIIa can be used in the treatment of hemophilia A and B. Factor IXa and factor VIIIa activate factor X. Factor VIIa eliminates the need for factors IX and VIII by activating factor X directly, and can overcome the problems of factor IX and VIII deficiencies with few immunological consequences.

The present invention is further illustrated by the following figures and examples, without being limited to these embodiments of the present invention.

### Figures:

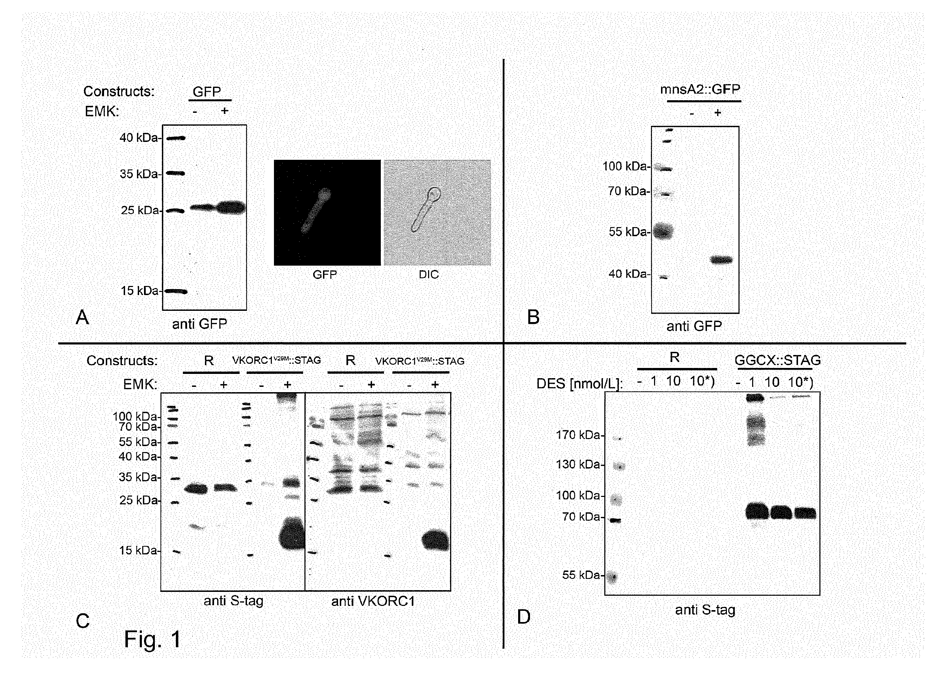

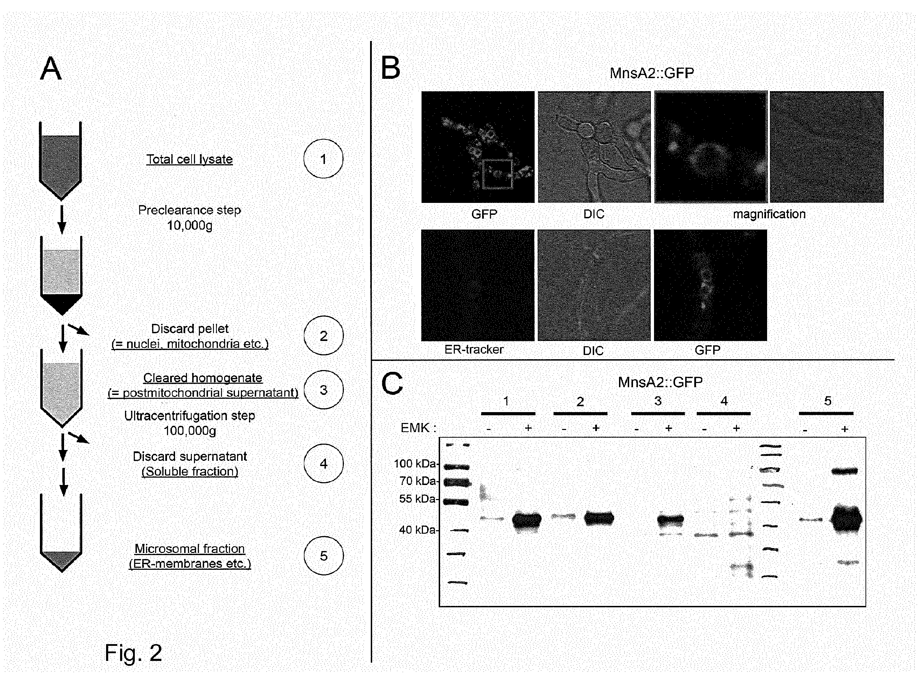

**Figure 1****:** Expression levels of GFP based control constructs, VKORC1^{V29M} and GGCX::Stag in total cell extracts. A) and B) Western blot of TCA precipitated total cell extracts of strains AN-GFP or AN-M2-GFP. Cultures were induced with 1 nmol/L DES when indicated (+) or left non-induced (-). Localization of GFP was analyzed by fluorescence microscopy.
   Microscopy data of mnsA2::GFP are shown in figure 2. C) Western analysis of TCA precipitated total cell extracts of AN-VmS and R (recipient strain = YR 23.5). Induction of cultures by 50 mmol/L EMK is indicated (+). Immunoblots were developed either by S-tag or by VKORC1 specific antibody. D) Western blot of TCA precipitated GGCX::Stag and R (recipient strain = YR 23.5) total cell extracts. Induction of cultures by DES is indicated (1,10) *). TCA preciptiated samples were extracted by a combination of Trition X-100 and SDS.
**Figure 2****:** Localization of MnsA2::GFP control construct. A) Preparation of fungal microsomes - Flow chart. The individual frations and supernatants are sequentially labelled. Cell fractionation was performed with all strains relevant for this study (Figure modified after (Abas and Luschnig, 2010)). B) Localization of MnsA2::GFP protein was analyzed by fluorescence microscopy. Red boxes indicate and show magnified images of the fungal hyphae. C) Cell fractionation and preparation of microsomes (final membrane pellet) of strain AN-M2-GFP. Western blots were performed to compare the individual fractions obtained during the preparation of microsomes. The individual fractions shown on the blot are sequentially labelled according to the flow chart A). Cultures were either induced with 50 mmol/L EMK (+), or left non-induced (-). Western blots were developed with GFP antibody.

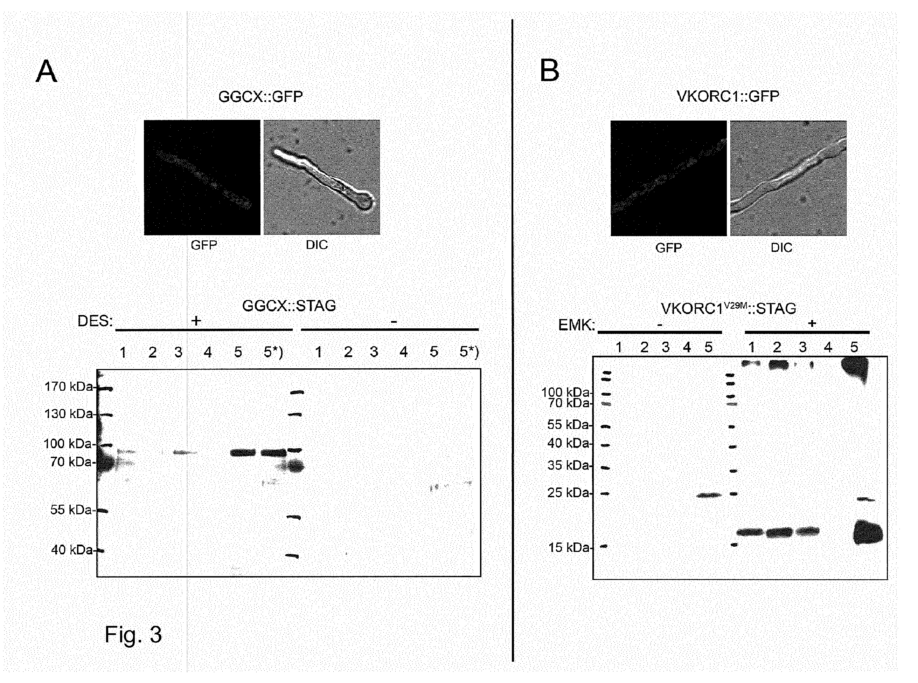

**Figure 3****:** Localization of GGCX::GFP (A) or VKORC1 **: :** GFP (B) protein was analyzed by fluorescence microscopy. Cell fractionation and preparation of microsomes (final membrane pellet) of strain AN-GS (A) and AN-VmS (B). Western blots were performed to compare the individual fractions obtained during the preparation of microsomes. The individual fractions shown on the blot are sequentially labelled according to Figure 2A. Cultures were either induced with 50 mmol/L EMK (+) or 1 nmol/L DES (+), or left non-induced (-). Both immunoblots were developed with S-tag antibody.

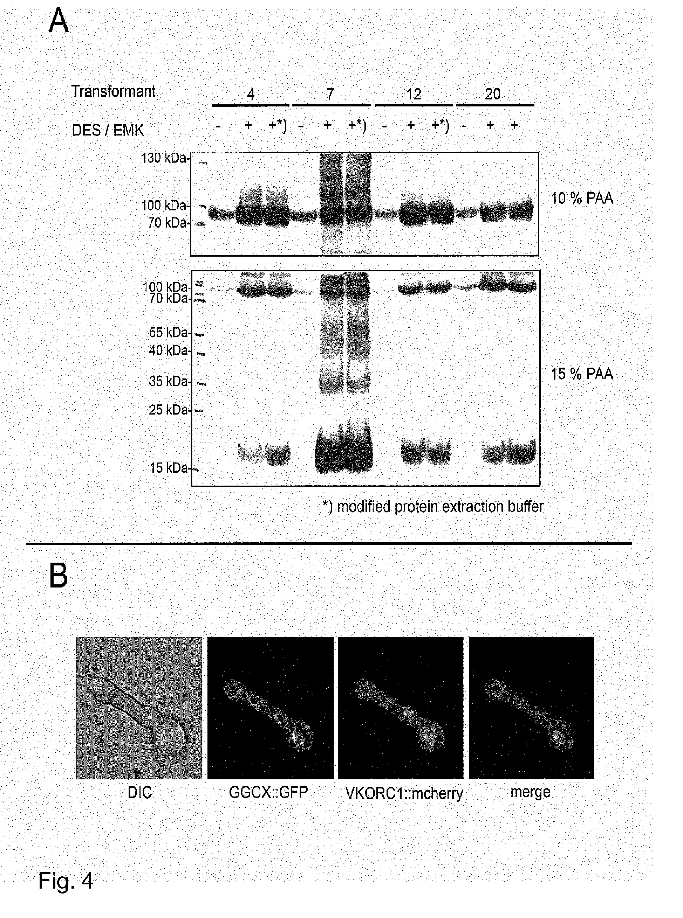

**Figure 4****:** A) Protein expression level of AN-VmS-GS transformants. Western blots of total cell extracts of a number of transformants, obtained after co-transformation of AN-VmS with plasmids pERE::GGCX::Stag and pgpdA(p)::hER are shown. *) indicates if samples extracted with a buffer additionally containing 1 % (v/v) Trition X-100 (see Materials and Methods). Total cell lysates were separated on a 10 % or a 15 % PAA-gel prior to Western blotting. Blots were developed with S-tag antibody. B) Localization of GGCX::GFP and VKORC1**: :**mCherry. Spores of strain AN-G**: :**GFP-V**: :**mCherry were grown overnight and induced for 4 h by EMK (50 mol/L) and DES (1 nmol/L). Z-stacked images obtained for each channel are shown.

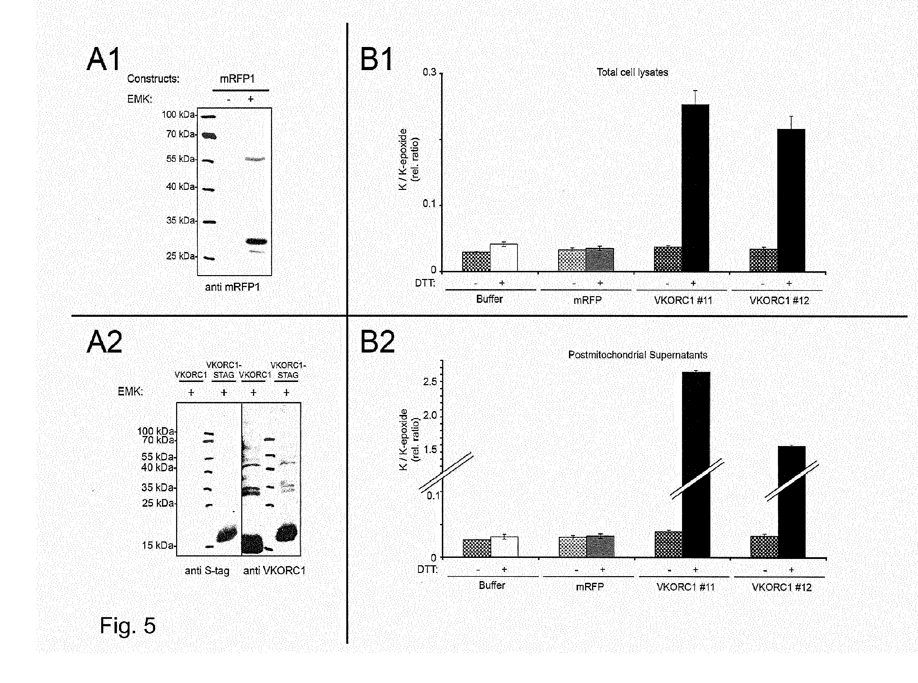

**Figure 5****:** VKORC1 in vitro activity assay. A1) and A2) Protein expression level of diverse VKORC1 and control constructs relevant for VKORC1 activity assay. Cultures were induced with 50 mmol/L EMK if indicated (+) or left non-induced (-). Total cell extracts of strains AN-RFP (A1), AN-VmS (A2) and AN-V (A2) were TCA-precipitated and analyzed by Western blotting. Blots were either developed with S-tag antibody, with VKORC1 specific antibody or with mRFP1 antibody. VKORC1 activity was assayed in total cell extracts (B1) and postmitochondrial supernatants (B2) Two transformants, AN-V 11, AN-V 12, obtained after transformation of YR 23.5 with plasmid pMT-V were analyzed for their vitamin K>0 reduction ability. Appropriate negative controls (AN-RFP, sample buffer) were included. B1) Fungal biomass was grown overnight and induced for 4 h by EMK (50 mol / L), disrupted by grinding in liquid nitrogen and further homogenized by sonification. Addition of DTT is indicated by (+). Incubation time was set to 240 min. B2) Postmitochondrial supernatants were taken for VKORC1 activity assays. Vitamin K>O concentration was set to 24 µmol / L and DTT was added when indicated. Vitamin K forms were analyzed by HPLC as already described and relative K / K>0 ratios were calculated. Error bars indicat standard deviation (total cell lysates n=5; PTM n=3).

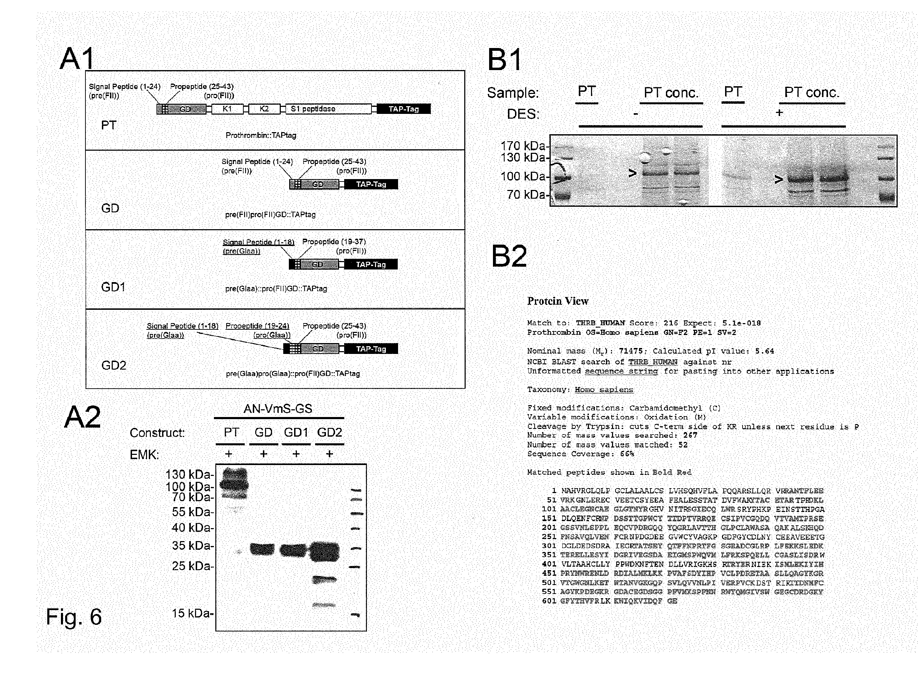

**Figure 6****:** A1) Overview gamma-carboxylation in vivo reporter constructs. Reporter constructs are based on human prothrombin (FII). Schematic representation of human prothrombin (GD = gamma carboxyglutamic acid domain, K1 and K2 = kringle domain, S1 peptidase = serin protease domain) For constructs GD1 and GD2 the pre and prepro peptide of GlaA (A.niger Glucoamylase) were fused to the FII GD as indicated. A2) Comparision of PT, GD, GD1 and GD2 expression. All cultures were grown in presence of 50 mmol/L EMK. Blots were developed with either calmodulin specfic antibody or with secondary HRP conjugated antibody (IgG specificity to Protein A of TAP tag). B1) MS analysis of spin-column concentrated prothrombin::TAP. Prothrombin:: TAP was expressed and purified as already described (Materials and Methods). Further concentration of prothrombin::TAP prior to MS-analysis was achieved via spin-columns. A colloidal coomassie stained PAA-gel of purified and concentrated prothrombin::TAP is shown. (+) indicates when prothrombin::TAP was purified from a culture grown in presence of DES (1nmol/L). B2) Mascot search results obtained for (heterologouly expressed, purified and concentrated prothrombin::TAP (PT conc.(+DES)).

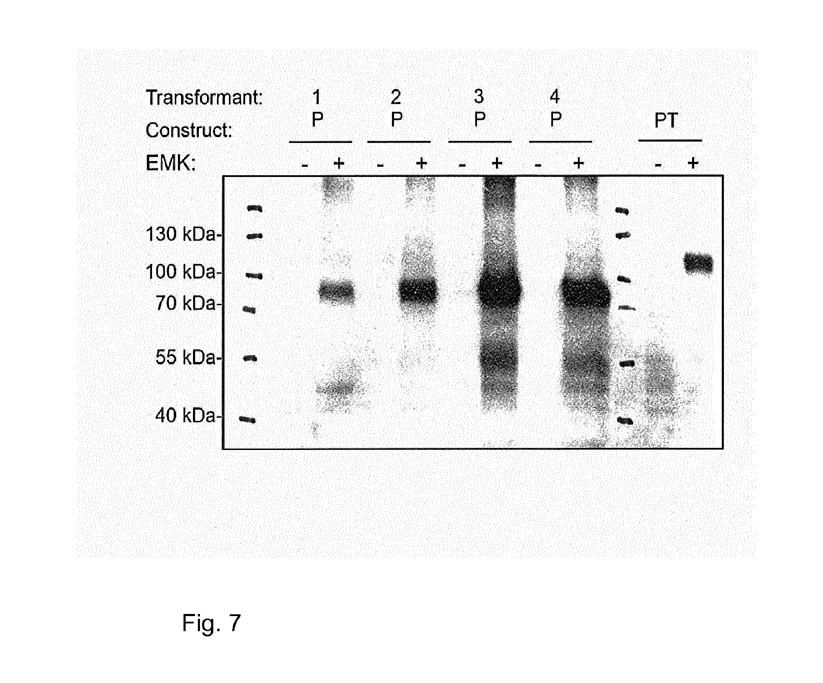

**Figure 7****:** Western Blots of total cell extracts of diverse P and PT transformants. PT = transformant expressing prothrombin::TAP, P = transformant expressing prothrombin. Recipient strain was AN-V. (+/-) indicates if cultures were induced (+) or not (-) with 50 mmol/L
   EMK. Blots were developed with prothrombin specific antibody (ERL).

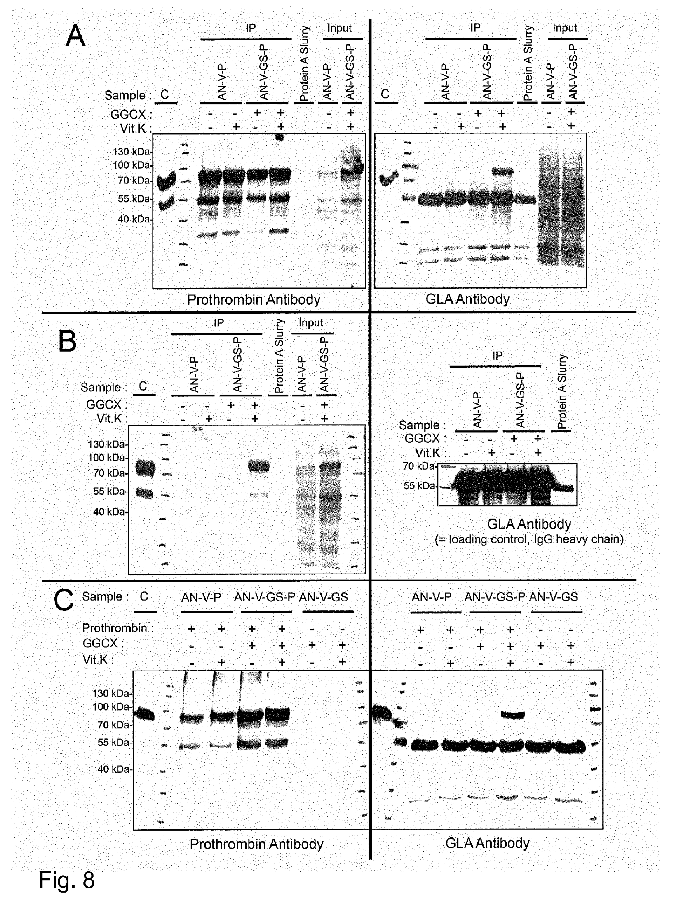

**Figure 8****:** Immunoprecipitation of prothrombin and analysis of posttranslational gamma-carboxylation. A) IP was performed with strain expressing VKORC1, GGCX, Prothrombin (AN-V-GS-P) and with strain expressing VKORC1 and Prothrombin (AN-V-P). All cultures were induced with EMK (50 mmol/L) and DES (1 nmol/L), Vitamin K addition is indicated by (+). IP was performed with prothrombin specific antibody (Hyphen). Western blots were developed with either prothrombin antibody (ERL) or a gamma-carboxylate glutamyl specific (gla) antibody (Brown et al., 2000). B) IP was performed with strain expressing VKORC1, GGCX, Prothrombin (AN-V-GS-P) and with strain expressing VKORC1 and Prothrombin (AN-V-P). All cultures were induced with EMK (50 mmol/L) and DES (1 nmol/L), Vitamin K addition is indicated by (+). IP was performed with gla specific antibody. Western blots were developed with prothrombin antibody (ERL). C) Prothrombin, expressed in AN-V-P or AN-V-GS-P strain was taken to perform IP. Strain AN-V-GS (does not contain prothrombin expression cassette) was used as another control. All cultures were induced with EMK (50 mmol/L) and DES (1 nmol/L), Vitamin K addition is indicated by (+). IP was performed with strain expressing VKORC1, GGCX, Prothrombin (AN-V-GS-P) and with strain expressing VKORC1 and Prothrombin (AN-V-P). Western blots were developed with either prothrombin antibody (ERL) or gla antibody. Input: Total cell lysates. IP: Final eluates of immunoprecipitated fungal expressed prothrombin; Protein A slurry: Incubation of Protein A slurry with Laemmli buffer, negative control. C Purified human prothrombin (ERL, ca. 175 ng).

### Examples:

### Example 1: Materials and media

### Aspergillus nidulans:

*A. nidulans* strain YR23.5 (Kainz et al. 2008) and derivatives thereof as modified herein were manipulated and grown in *Aspergillus* minimal medium (MM) according to (Pontecorvo et al., 1953; Hill et al.), with appropriate supplements and diammoninum tartrate (10 mM) as nitrogen source. Generally, 1 % (w/v) glucose was added to MM as carbon source (GMM); for protein expression media 0.2 % fructose (w/v) was the sole carbon source (FMM) to prevent carbon repression of alcA promoter. For VKORC1 expression *A. nidulans* strains were pre- grown for 14 h overnight at 37°C, shaking at 180 rpm in 1 L Erlenmeyer flasks containing 250 ml of FMM. Cultures were either induced for 4 h by 50 mmol/L EMK (Ethylmethylketone, Sigma) or left non-induced, as negative control. The mycelium was filtered, squeezed between paper towels to remove excess of liquid and frozen in liquid nitrogen. The frozen mycelium was either processed immediately or stored at -80°C.
*E.coli :* Subcloning Efficiency DH5-Alpha™ Competent Cells (Invitrogen) were used for plasmid propagation. Transformation procedure was done according to manufacturers' manual. For of blue/white selection 400 µg/mL Xgal and 40 µg/mL IPTG was added to the media.

### Example 2: Vitamin K growth tests

Solid Media. Minimal media plates including vitamin K (vitamin K1, Fluka, 99%) were prepared. On top of the agar medium, sterile cellophane was placed to inhibit direct contact between the media and fungal cells. Spores of strain YR23.5 were plated in a concentration of ca. 10E+5 per plate. After 3 days of growth, harvesting was done by scraping off the mycelium from cellophane. Then the mycelium was frozen and pulverized in liquid nitrogen. The powder was subsequently lyophilised and sent for vitamin K analysis to LUFA-ITL (Landwirtschaftliche Untersuchungs- und Forschungsanstalt Institut für Tiergesundheit u Lebensmittelqualität GmbH, Kiel, Germany). Liquid media. The strain YR23.5 was pre-cultivated for 48 hours in 2 x 250 ml of liquid minimal media at 37°C, 180 rpm in Erlenmeyer flasks. Mycelium was harvested by filtration, washed and re-suspended in sterile minimal medium and separated into four equal aliquots of roughly 250 mg wet weight (corresponding to roughly 50 mg dry weight). Culture volume was set to 30 ml. Two of these cultures received different vitamin K amounts, final concentration: 0.5 mg/mL and 0.05 mg/mL vitamin K (Konakion, micelles suspension, Roche, 10 mg/mL) and were grown for further 8 hours. Mock controls were grown in parallel for 8 hours without receiving vitamin K. After the growth phase the fungal cells were inactivated by boiling in the microwave oven (3 x 5 min at max. energy level), and subsequently vitamin K was added to the mock controls (final concentration, 0.5 and 0.05 mg/mL vitamin K). The inactivated cultures were further incubated in the rotary shaker for 30 minutes. All cultures were harvested at the same time point. Harvested biomass was washed, frozen and ground in liquid nitrogen, lyophilised and sent for analysis to LUFA-ITL (Landwirtschaftliche Untersuchungs- und Forschungsanstalt Institut für Tiergesundheit u Lebensmittelqualität GmbH, Kiel, Germany).

### Example 3: Cloning of VKORC1 constructs

For cloning of VKORC1 expression and mRFP control constructs pMT-mRFP1 (Toews et al., 2004) served as basis vector. pMT vectors comprise the fungal alcA promoter, argB (auxotrophic marker), bla (ampicillin-resistence gene). pERE based constructs are under the control of the EREuraRS promoter and are lacking a fungal auxotrophic marker sequence. All PCR reactions were performed using Phusion TM Flash High-Fidelity PCR Master Mix (Finnzymes), Pwo Polymerase (Roche) or HiFi-PCR (Fermentas). Plasmids were sequenced prior to *A.nidulans* transformation to ensure that no mutations occurred during the PCR amplification steps. Control constructs pMT-mRFP. From pMT-mRFP1 (Toews et al., 2004) the mRFP coding sequence was amplified using oligonucleotides mRFP_BamHI_fwd and mRFP_KpnI_rev. The fragment was subcloned into pGEM-Teasy. After sequencing, mRFP1 coding sequence was obtained by BamHI/KpnI excision and ligated into the BamHI/KpnI digested pMT-mRFP1 backbone. pMT-mnsA2::mRFP1. MnsA2 sequence was amplified with primer mnsA-1 BgII fwd and mnsA2_EcoRI_rev using plasmid pAN52_mnsA-2_GNT II as template (Kainz, 2006). The PCR fragment was subcloned into pGEM-Teasy, resulting in mnsA-2 pGEM-Teasy. The leader sequence was excised from mnsA-2_pGEM-Teasy and cloned into BamHI/EcoRI restricted pMT-mRFP1. pERE: :sGFP. EREuraRS promoter was amplified from plasmid p2059-URA-RS with primers ERE_ura_RS_Bgl_II_fwd and ERE_ura_RS_PacI_rev and subcloned into pGEM-Teasy, resulting in EREuraRS_pGEM-Teasy. sGFP was amplified from plasmid pMT-sGFP (Toews et al., 2004) with primers GFP_NotI_rev and GFP_PacI_fwd and subcloned into pGEM-Teasy. The resulting plasmid was digested by NotI and PacI for release of the GFP fragment. The PacI/NotI GFP fragment was ligated together with a BglII/PacI EREuraRS fragment into the BglII/NotI digested plasmid p2059-URA-RS. (Pachlinger et al., 2005) pthiA: :hER: :trpC. (human estrogen receptor) was vector designed in our lab by Lisa Olsen (Olsen 2006). In this plasmid the *Aspergillus oryzae* thia promoter is inserted upstream the hER ORF.
pERE-mnsA2::GFP. MnsA-2 sequence was amplified with primer mnsA-1_PacI_fwd and mnsA-2_BamHI_rev using plasmid pAN52_mnsA-2_GNT II as template (Kainz, 2006) GFP was amplified with primers GFP_NotI_rev and GFP_BamHI_fwd from plasmid pMT-sGFP and subcloned into pGEM-Teasy. The BamHI/NotI digested GFP fragment, together with the PacI/BamHI digested mnsA-2 fragment was inserted into the PacI/NotI digested plasmid pERE::sGFP.

### VKORC1 expression constructs.

The nucleotide sequence of human VKORC1 cDNA (Vitamin K epoxide reductase complex subunit 1 UniProt identifier: Q9BQB6-1) was codon-optimized for improved translation in *A.nidulans.* Codon-usage tables of http://www.kazusa.or.jp/codon/ were taken to perform the optimization with a Microsoft Excel script. VKORC1 was synthesized with mutation of nucleotide 85 (G85A) and a carboxy-terminal S-tag sequence, named VKORC1^{V29M}: :Stag. The synthesized fragment was cloned into pUC57 vector by Genescript USA Inc. New Jersey. pMT-VKORC1^{V29M}: :Stag. VKORC1^{V29M}: :Stag was inserted by BamHI (5') and KpnI (3') into pUC57 vector. The BamHI/KpnI VKORC1^{V29M}: :Stag fragment was cloned into the BamHI/KpnI digested backbone of pMT-mRFP1. *A.nidulans* trpC (anthranilate synthase component 2, AN0648) terminator was amplified with primers KpnI_trpC_fwd and trpC_KpnI_rev. The resulting PCR product was digested with KpnI and ligated into the KpnI linearized and dephosphorylated plasmid. pMT-mnsA2: :VKORC1^{V29M}: :Stag. MnsA2 leader was amplified with primers mnsA1_BgII_fwd
andmnsA2_NdeI_BamHI_rev. PCR product was digested with BglII and BamHI. The mnsA2 fragment was cloned into BamHI linearized pMT-VKORC1^{V29M}: :Stag plasmid. Due to the ligation of a BglII with a BamHI site, this restriction site was not accessible anymore for further cloning purposes. pMT-mnsA2: :VKORC1^{V29M} (28-163): :Stag. The plasmid was constructed by NdeI digestion of pMT-mnsA2: :VKORC1^{V29M}: :Stag. A 84 bp fragment, representing the amino terminal part of VKORC1 was cut out of the vector. The remaining backbone was ligated, resulting in pMT-mnsA2: :VKORC1^{V29M} (28-163)::Stag. pMT-VKORC1. Reversal of G85A mutation of pMT-VKORC1^{V29M}: :Stag and removal of S-tag was achieved by PCR. A small synthesized DNA fragment, namely VKOR_mut_29_F of ca. 120 bp was used as forward primer and VKOR_rev_STOP_EcoRI was used as reverse primer. The resulting VKORC1 fragment was digested with BamHI and EcoRI. TrpC terminator was amplified with primers Eco-RI_trpC fwd and trpC_KpnI_rev. The resulting trpC fragment was EcoRI and KpnI digested. Finally pMT_mRFP vector was cut with BamHI and KpnI and the VKORC1 and trpC fragments were inserted by ligation. pMT-VKORC1: :Stag. The pMT-VKORC1: :S-tag was cloned according to the protocol described for pMT-VKORC1, with minor modification: Reverse primer VKOR_rev_S-tag_STOP_EcoRI instead of VKOR_rev_STOP_EcoRI was used for initial amplification and mutation of the VKORC1 fragment.

### Example 4: Aspergillus transformation

Transformation protocols basically followed the procedures published by Tilburn and colleagues (Tilburn et al., 1983). Strains, harbouring constructs under the control of the EREuraRS promoter were co-transformed with plasmid pthia::hER (Olsen, 2006). Transformants were selected according to the auxotrophic marker and analyzed by PCR. Southern blotting and hybridization was performed as described by Sambrook, briefly: Genomic DNA was digested into fragments with appropriate restriction enzyme(s) and then separated on a 0.8 % (w/v) agarose gel. Afterwards the DNA was denaturated and transferred to HYBOND N+ membrane (Amersham) by capillary blotting. Probes were amplified from plasmid DNA by using primers Epox_new_fwd and VKORC1_opt_rev. ³²P-labelling of probes was done with Ready-To-Go DNA Labelling Beads (GE Healthcare). Membranes were exposed to a Storage Phosphor Screen (GE Healthcare).

### Example 5: Transcriptional analysis

RNA isolation. Strains were grown overnight on fructose minimal medium (FMM) containing the appropriate supplements at 37°C and 180 rpm and induction of alcA promoter was achieved by EMK (final concentration 50 mmol/L) for 120 - 240 min. The mycelium was harvested and frozen in liquid nitrogen. Mycelium was ground to fine powder and RNA was isolated using Trizol™ (Invitrogen) according to manufacturers' instructions. Transcriptional analysis was either performed by qRT-PCR or Northern Blots. qRT-PCR:cDNA synthesis was performed with SuperScript® III First-Strand Synthesis Kits (Invit-rogen) or IScript™ cDNA synthesi kit (Biorad) according to manufacturer's instructions. Quantitative PCR (qRT-PCR) for gene expression analysis was performed with Platinum Sybr green qPCR SuperMix-UDG (Invitrogen) or SYBR™ Green Super Mix (Biorad) and was carried out on an iCycler thermal device with a MyiQ single-colour real-time PCR detection system (Bio-Rad). Reactions were run in duplicate, and a mean value of the two samples was calculated. The results were either calculated by the standard curve method or by the Pfaffl method (Pfaffl, 2001), both methods referring to actin as internal standard. For the standard curve method, the relative amount of PCR product were adjusted for the level of internal control amplification (actin mRNA), which was obtained from the same sample. Northern blot hybridizations were carried out as described previously. (Gómez et al., 2003; Reyes-Dominguez et al., 2008) Total RNA extracts of *A.nidulans* were denaturated with Glyoxal (Sigma). The denaturated RNA samples were separated by electrophoresis on agarose gels. After electrophoresis, RNA was blotted on a nylon membrane via capillary transfer overnight. Membranes were hybridized with ³²P labeled probes after UV-crosslinking. Probes were amplified from plasmid DNA by using primers Epox_new_fwd and VKORC1_opt_rev. ³²P labelling of probes was done with Ready-To-Go DNA Labelling Beads (GE Healthcare). Membranes were exposed to a Storage Phosphor Screen (GE Healthcare) scanned with Storm 860 imager (GE Healthcare) and analyzed with ImageJ. (Rasband, 2011).

### Example 6: Total cellular protein extraction

Mycelium was harvested, squeezed, frozen and manually ground in liquid nitrogen. The powder was suspended in 800 µL of ice cold buffer E (50 mmol/L TRIS pH 7, 150 mmol/L NaCl, 2 mmol/L EDTA, including Fungal Protease Inhibitior cocktail (Sigma)). Once homogenized by vortexing, 200 µl solution T (50 % (w/v) TCA (Roth) and 0,2 % (w/v) sodium deoxycholate (Sigma)) was added and mixed. The precipitate was incubated on ice for 30 min. Afterwards samples were centrifuged at 13.000 x g for 10 min. The pellet was care-fully washed once with 1 ml ice cold ethanol and once with ice cold acetone. Between the washing steps the samples were centrifuged for 5 min at 13.000 x g. The acetone was re-moved, the protein pellet was air-dried and subsequently dissolved in buffer L (50 mmol/L TRIS pH 7.5, 4 % (w/v) SDS, 75 mmol/L NaCl, including Protease Inhibitor Cocktail). Samples were incubated for 60 min at 30°C on a thermo-shaker. To remove un-dissolved cell debris, centrifugation for 10 min at 13.000xg was performed and supernatants were taken. Protein concentration in the supernatant was measured using the BCA protein quantification kit (Pierce, USA). Total protein extractions were mixed in the ratio 3:1 with Laemmli buffer, including 0.1 % (w/v) bromophenol blue and 400 mol/L DTT, 30 % (v/v) glycerol. Samples were analyzed with standard SDS-PAA gel electrophoresis on 10, 12 or 15 % PA-gels. Western Blots were performed according to Towbin et al. (1979) on a Mini Trans-Blot System (Biorad). For immunoblots, membranes were hybridized with alpha-RFP antibody (US Biological, Cat No R1310), alpha- GFP antibody (ROCHE), polyclonal S-tag antibody (Abcam) or custom-made VKORC1 antibody.

### Example 7: Confocal fluorescence microscopy (CFM)

Strains were grown on cover slips in GMM containing 1 % of glucose and appropriate supplements. Induction of proteins driven by the EREuraRS promoter was done 4h prior to microscopy by the addition of 1 nmol/L diethylstilbestrol (DES, Sigma) to the media. Microscopic observations were performed with a Leica TCS SP2 confocal laser scanning microscope. GFP was excited at 488 nm (Ar-Kr laser) and emission was measured be-tween 500 and 555 nm. 4',6-diamidino-2-phenylindole (DAPI) was excited by a short arc mercury lamp and emission was detected between 426 and 479 nm. Nuclei were stained with DAPI as previously described (Berger et al., 2006). ER-Tracker Blue-White DPX (Invitrogen) staining was performed as described by Watanabe et al. (2007).

### Example 8: Purification of VKORC1 antibody

A synthetic peptide corresponding to amino acid 151-163 of VKORC1 (C-RKVQEPQGK-AKRH-N(14aa)) was conjugated to KLH (Keyhole limpet hemocyanin) carrier protein and taken for immunization of two rabbits. Peptide synthesis and immunization of animals was performed by Biogenes GmbH, Germany. Affinity purification of antibodies was either per-formed according to a protocol developed by Michael Koelle
(http://130.15.90.245/antibody_purification.htm) or according to PoorMans' protocol (S. Goller personal communication). The purification procedure was performed in our lab and the critical steps of the protocol are outlined: Two milligrams of the KLH conjugated peptide were spotted on a nitrocellulose membrane and used for affinity purification of the polyclonal VKOR-antibody. Next, the dried membrane was incubated on a shaker for 1 h in TBS (TRIS-buffered saline) / 1 % (w/v) BSA (bovine serum albumin). After washing the membrane with TBS, 10 ml of serum was added and incubated on a shaker for at least 1 h. The membrane was washed several times, with TBS-T (TBS containing 0,1 % (v/v) Tween-20 (Sigma)) and TBS. Elution of the bound antibody was achieved by adding glycine (100 mmol/L, pH 2.5) and vigorous shaking for 1 min. The 'low pH' antibody-glycine solution was quickly neutralized with NaOH (1 mol/L). Furthermore BSA (final con centration 1 % w/v) and NaN₃ (0.05 % w/v) was added for stabilization. Semi-quantitative immunoblotting was performed for evaluation of the antibody and the purification process.

### Example 9: Microsomal preparations

Preparation of rat liver microsomes. Male Sprague Dawley rats were sacrificed by de-capitation; livers were extracted, rinsed with buffer (150 mM KCl, 50 mM Tris, pH 7.4), minced with scissors, and homogenized on ice in a motor-driven Potter-Elvehjem homog-enizer with three volumes of buffer. The crude homogenate was centrifuged at 8750 x g for 15 min at 4°C. Microsomes were sedimented from the 8750 x g supernatant by centrifugation at 165,000 x g for 38 min at 4°C. After two washing steps, microsomes were dispersed in homogenizing buffer to provide a protein concentration of approximately 30 mg/ml. (done in the lab of Prof. Lars Gille.) Preparation of fungal microsomes Mycelium (after squeezing between paper towels) was ground in liquid nitrogen to very fine powder and re-suspended and mixed in the ap-propiate disruption buffer by vortexing. After incubation on ice for 5 minutes cell debris was pelleted at 8.000 x g for 10 minutes at 4°C. The supernatant was subsequently transferred into an ultra centrifugation tube and microsomal fraction was pelleted at 120.000 x g for 90 minutes (4°C). The microsomal pellet was re-suspended in buffer and stored at -80°C. The different fractions obtained by preparation of fungal microsomes were analyzed by Western blotting. Proteins of the fractions were either directly extracted by SDS-extraction buffer or were TCA precipitated prior to extraction. Protein concentration in the supernatant was determined using the BCA protein quantification kit (Pierce).

### Example 10: VKORC1 activity assays

Vitamin K epoxide was prepared from vitamin K1-quinone by regioselective epoxidation with hydrogen peroxide according to the method of Tishler (Tishler et al., 1942). Vit. K epoxide was synthesized in 99,8% purity and was used for in vitro VKOR activity assays. Prior to each VKOR activity assay purity of Vitamin K epoxide was verified by HPLC. Vit K epoxide was diluted in DMSO. Setup of the in vitro assay: For crude cell extracts, mycelium, which was frozen in liquid nitrogen was manually ground to fine powder and re-suspended in Buffer T100, T50, T35 or SI. T100: 100 mmol/L Tris (pH 7,5 at RT), Saccharose 250 mmol/L, KCl 50 mmol/L, 0,60 % (v/v) Protease Inhibitor Mix, DTT 5 mmol/L (if required); T50: like T100 but 50 mmol/L Tris (pH 7,5 at RT); T35: like T35 but 35mmol/L Tris (pH 7,8 at RT); SI: 25mmol/L Imidazol (pH 7,8 at RT) 25 mmol/L Sucrose, 0,5 % (v/v) CHAPS, 0,6 % (v/v)Protease Inhibitor Mix, DTT 5 mmol/L (if required).
After mixing by vigorous vortexing, the total cell lysates were stored at 4°C. When indicated, cell lysates were further homogenized by sonification with Diagenode™ Bioruptor in 50 ml tubes. A stopper with a metallic bar to reflect sound waves was used to improve resonance efficiency in the tubes. Postmitochondrial supernatants were generated by clearing crude cell extracts by a centrifugation step of 5000 x g. A.nidulans and rat liver microsomes were prepared as described above. The frozen microsomes were resuspended in the appropriate buffer prior to VKORC1 assays. Protein concentration in the supernatant was quantified using the BCA protein quantification kit of Pierce. Sample volumes of 200 µl or 1500 µl containing total protein concentrations in a range of 1.5 to 5 mg/ml were incubated in the buffer indicated. Samples were agitated on a thermo-shaker in the dark with an incubation temperature set to 30 °C. Reaction was started by addition of Vit.K Epoxide to a final concentration of 24 or 40 µmol/L. After 30 min - 240 min incubation time, samples were snap frozen in liquid nitrogen. Prior to HPLC analysis (Prof. Lars Gille), samples were thawed and SDS was added to a final concentration of 50 mmol/L prior to extraction with 1 mL hexane/ethanol (5:2 v/v). After 5 min vortexing the samples were centrifuged for 5 min at 9000 x g. The upper organic layer was removed and evaporated by a stream of argon. The residue was dis-solved in 120 µl mobile phase (without electrolyte) and 100 µl were transferred to HPLC mini sample vials. HPLC analysis was performed on a Waters LC1 module with an internal UV detector and a coulometric detector Coulochem III (ESA) equipped with a conditioning cell (5021A) and an analytical cell (5010A, E1 upstream electrode, E2 downstream electrode). The injection volume was 40 µL. Vitamin K-related compounds were eluted from a reversed phase column (Hibar RT 125-4, LiChrospher 100 RP-18 (5 µm)) combined with a pre-column (LiChroCART 4-4, LiChrospher 100 RP-18 (5 µm)) with 41 mM NaClO₄ in ethanol/ methanol/ acetonitrile/ HClO₄ (240 : 400 : 300 : 0.5) at 1 mL min-1 (25 °C). The conditioning cell between column and detector was set to -600 mV and E1 of the analytical cell was set to -600 mV for post-column reduction. Vitamin K1 was detected coulometrically in the oxidative mode using the analytical cell of the ESA detector (E2 = +450 mV, gain 10 µA). Vitamin K1 epoxide was detected by the UV absorption at 266 nm. Typical elution times for vitamin K1 epoxide and vitamin K1 were 5 and 8 min, respectively. The peak area ratio for vitamin K1 to vitamin K1 epoxide was calculated from the respective peak areas in the ECD (8 min) and UV (5 min) chromatogram. This ratio was determined for each sample type from three, five or seven independent incubations.

### Example 11: Cloning of GGCX constructs

pERE-GFP. EREuraRS promoter was amplified from plasmid p2059-URA-RSwith primers ERE_ura_RS_Bgl II_fwd and ERE_ura_RS_PacI_rev and subcloned into pGEM-Teasy, resulting in EREuraRS_pGEM-Teasy. sGFP was amplified with primers GFP_NotI_rev and GFP_PacI_fwd and subcloned into pGEM-Teasy. The resulting plasmid was digested by NotI and PacI for release of the GFP fragment. The PacI/NotI GFP fragment was ligated together with a BglII/PacI EREuraRS fragment into the BglII/NotI digested plasmid p2059-URA-RS. pERE-GGCX::Stag. GGCX (uniprot: P38435 (VKGC_HUMAN)) was codon-optimized for expression in As*pergillus nidulans* according to codon usage database (http://www.kazusa.or.jp/codon/). The one amino acid - one codon approach http://genomes.urv.es/OPTIMIZER/ was used for optimization. The codon-optimized nucleic acid sequence was synthezied by Mr. Gene Inc. (Germany, now Invitrogen) containing a PacI and BamHI site on 5' end and a NotI site on its 3' end. The fragment was cloned into a pMS (spectinomycin resistance cassette) vector backbone resulting in pMS::GGCX. The resulting plasmid was digested with PacI and NotI. The released GGCX fragment was ligated into PacI and NotI digested plasmid pERE-GFP. The resulting plasmid was named pERE-GGCX::Stag. pERE-mnsA2::GGCX::Stag. MnsA-2 sequence was amplified with primer mnsA-1_PacI_fwd and mnsA_2_BamHI_rev using plasmid pAN52_mnsA-2_GNT II as template (Kainz, 2006). The PCR fragment was subcloned into pGEM-Teasy, resulting in PacI_mnsA-2_BamHI_pGEM-Teasy. The leader sequence was excised from PacI_mnsA-2_BamHI_pGEM-Teasy and ligated into PacI and BamHI digested pERE::GGCX::Stag. The resulting plasmid was named pERE-mnsA2::GGCX::Stag. gpdA(p): :hER: :trpC(t): :riboB was taken from Pachlinger et al., 2005.

### Example 12: Cloning of Prothrombin constructs

p-alcA(p)::prothrombin::TAP. Human P00734 (THRB_HUMAN) was codon-optimized for expression in *A. nidulans* according to codon usage database (http://www.kazusa.or.jp/codon/). The one amino acid - one codon approach (http://genomes.urv.es/ OPTIMIZER/) was used for optimization. Sequence was further optimized manually, mainly by elimination unfavourable GC stretches. However, the amino acid sequence was not changed. The codon-optimized nucleic acid sequence was synthesized by Mr. Gene Inc. (Germany, now Invitrogen) containing a PacI site on 5' end and the nucleotide sequence of 5xGA (1 letter aa code) followed by a NcoI restriction site on its 3' end. The sequence was cloned into a pMS (spectinomycin resistance cassette) vector backbone resulting in pMS::prothrombin. The vector was digested with PacI and NcoI and a fragment of 1900 bp, representing prothrombin was released. A 1200 bp fragment comprising C-TAP (Puig et al., 2001) and *Aspergillus nidulans* trpC terminator was excised with NcoI and XhoI from vector pGem-Teasy: :alcA(p): :C-TAP: :trpC(t) (Bayram et al. 2008, T. Schinko, unpublished). alcA(p) was amplified from plasmid DNA of pGem-Teasy: :alcA(p): :C-TAP: :trpC(t) using chimeric primers KpnI_BglII_palca_F and palca_PacI_NcoI_R. The obtained PCR product was digested with KpnI and NcoI. The digested prothrombin fragment, the C-TAP: :trpC(t) fragment were cloned into the KpnI and XhoI digested pBluescript SK II (+) backbone, resulting in pBlue: :alcA(p): :C-TAP: :trpC(t). Aspergillus nidulans pyrG gene was amplified using primers BglII_pyrG(An)_F and pyrG(An) R. The obtained 3 kb fragment was BglII digested and cloned into the BglII linearized vector pBlue::alcA(p)::C-TAP::trpC(t), resulting in vector pBlue:3pyrG(An)5::alcA(p)::C-TAP: :trpC(t). The PacI and NcoI digested prothrombin::5xGA fragment was ligated into pBlue:3pyrG(An)5: :alcA(p): :C-TAP: :trpC(t) digested with the same enzymes to give vector p-alcA(p)::prothrombin::TAP. p-alcA(p)::preproGD(prothrombin)::TAP. preproGLA(Prothrombin) was amplified from plasmid DNA of vector pMS::prothrombin with chimeric primers PacI_proproGLA_F and proproGLA_5×GA_NcoI_R. A 5xGA (glycine, alanine) spacer was introduced with the reverse primer. The PCR product was digested with NcoI and PacI and ligated into pBlue:3pyrG(An)5: :alcA(p): :C-TAP: :trpC(t) vector digested with the same enzymes. p-alcA(p): :pre(GLAA): :proGD(prothrombin): :TAP. prepro-GLA(Prothrombin) was amplified from plasmid DNA of vector pMS::prothrombin with chimeric primers G1-gla_F and proproGLA_5xGA_NcoI_R. A 5xGA (glycine, alanine) space was introduced with the reverse primer. The PCR product was digested with NcoI and PacI and ligated into pBlue:3pyrG(An)5::alcA(p)::C-TAP: :trpC(t) vector digested with the same enzymes. p-alcA(p)::prepro(GLAA)::proGD(prothrombin)::TAP. prepro-GLA(Prothrombin) was amplified from plasmid DNA of vector pMS::prothrombin with chimeric primers G2-gla_F and propro-GLA_5xGA_NcoI_R. A 5xGA (glycine, alanine) space was introduced with reverse primer. The PCR product was digested with NcoI and PacI and ligated into pBlue:3pyrG(An)5: :alcA(p): :C-TAP: :trpC(t) vector digested with the same enzymes. p-alcA(p)::preproGD(prothrombin). Aspergillus nidulans trpC terminator was amplified with primers NcoI_trpc_fwd and trpc_NotI_rev from plasmid palcA(p)::preproGD(prothrombin)::C-TAP and the resulting PCR product was digested with NcoI and NotI. prepro-GLA(Prothrombin) was amplified from vector pMS::prothrombin with primers PacI_pro_fwd and Pro_STOP_NcoI_rev. The PCR product was digested with NcoI and PacI and ligated together with the NcoI and NotI digested trpC(t) fragment into
pBlue:3pyrG(An)5: :alcA(p): :C-TAP: :trpC(t) vector digested with NcoI and PacI. p-gpdA(p)::GGCX. A 1,8 kb fragment comprising gpdA promoter was amplified from genomic A. nidulans DNA with primers PacI_gpdA_fwd and BamHI_gpdA_rev. The PCR product was digested with PacI and BamHI. Aspergillus fumigatus riboB gene was amplified fromplasmidDNAwith primers ClaI_riboB_35_fwd and riboB_35_PacIX_BamHI_rev. The obtained PCR product was digested with ClaI and PacI. GGCX was amplified with primers BamHI_GGCX_fwd and GGCX_NotI_rev. The PCR product was digested with BamHI and NotI. Vector pERE::GGCX::Stag was digested with ClaI and NotI. The vector backbone was gel purified and ligated with riboB, gpdA, GGCX fragments (digested with enzymes as mentioned above). The resulting vector was named pgpdA(p)::GGCX. p-gpdA(p)::GGCX::S-tag. GGCX::S-tag fragment was excised from vector pERE: :GGCX with enzymes BamHI and NotI. The fragment was subsequently cloned into pgpdA(p)::GGCX, where GGCX(without tag) was excised with BamHI and NotI.

### Example 13: TAP purification

The protocol of TAP purification is based on a protocol of University of Washington
(http:\\depts.washington.edu/yeastrc/pages/ms-tapl.html) and (Bayram et al., 2008). However, several steps were modified and are outlined as follows: Mycelium was harvested in frozen in liquid nitrogen. Mycelia were ground in liquid nitrogen to prepare total celllystes in Buffer LP (100 mmol/L TRIS-HCl pH 8, 300 mmol/L NaCl, 10 % Glycerol, 0,05 % CA-630 (Sigma), 2 mmol/L EDTA, 2 mmol/L DTT and protease inhibitors (complete EDTA-free (Roche), aprotinin (Roth), E-64 (Sigma), PMSF (Roche)). Protease inhibitors were used in concentrations according to references and manufacturers' recommendations. Cell lysates were vigorously mixed by vortexing and centrifugated at 15.000 x g for 20 minutes. Approximately 10 ml of supernatants were transferred to 15 ml tubes and 170 µl IgG sepharose 6 Fast Flow (Amersham) was added. The extracts were incubated on a rotary shaker for at least 200 minutes. The cell lysate / IgG sepharose suspension was poured onto a gravity-flow chromatography column (Poly-Prep, Biorad). The cell lysates were passed through the settled IgG sepharose by gravity. Sepharose was washed 2 x 10 ml with IPP 300 (25 mmol/L TRIS-HCl pH 8, 300 mmol/L NaCl, 0,1 % (v/v) NP-40, 1 mmol/L DTT containing protease inhibitors (complete EDTA-free (Roche), aprotinin, pepstatin A, E-64). Next the sepharose was washed 1 x 10 ml IPP 150 (25 mmol/L TRIS-HCl pH 8, 150 mmol/L NaCl, 0,1 % (v/v) NP-40, 1 mmol/L DTT and protease inhibitors (complete EDTA-free (Roche), aprotinin, pepstatin A, E-64)) and 1 x 10 ml with TEV CB (25 mmol/L TRIS-HCl pH 8, 150 mmol/L NaCl, 0,1 % (v/v) NP-40, 1 mmol/L DTT, 0,5 mmol/L EDTA and protease inhibitors (pepstatin A, E-64) The bottom of the column was closed with a stopper. TEV cleavage was performed under rotation using 250 U of ProTEV (Promega) at 4°C over night. The TEV eluate was drained into a sealed Polyprep column and the previous column (column used for TEV cleavage) was additionally washed with 1 ml TEV CB. Three volumes of CBB-01 (25 mmol/L TRIS-HCl, 150 mmol/L NaCl, 0,1 % (v/v) NP-40, 1 mmol/L Magnesium acetate, 1 mmol/L Imidazole, 2 mmol/L CaCl₂, 10 mmol/L b-mercaptoethanol and protease inhibitors (complete EDTA-free (Roche), pep-statin A, E-64, PMSF) was added plus 3 µl of 1 mol/L CaCl₂ per mL of IgG eluate. In addition, 300 µl of calmodulin Sepharose 4B (Amersham) were equilibrated in CBB-01 and added. Finally, the column was sealed and incubated on a rotary platform for 120 min. The settled calmodulin sepharose was washed 2 x 1 mL CBB-01 and 1 x 1 mL CBB-002 (25 mmol/L TRIS-HCl, 150 mmol/L NaCl, 0,02 % (v/v) NP-40, 1 mmol/L Mag-nesium acetate, 1 mmol/L Imidazole, 2 mmol/L CaCl₂, 10 mmol/L b-mercaptoethanol and protease inhibitors (complete EDTA-free (Roche), pepstatin A, E-64, PMSF). The bottom of the column was plugged and 1 mL of CEB (25 mmol/L TRIS-HCl, 150 mmol/L NaCl, 0,02 % (v/v) NP-40, 1 mmol/L Magnesium acetate, 1 mmol/L Imidazole, 20 mmol/L EGTA, 10 mmol/L b-mercaptoethanol and protease inhibitors (complete EDTA-free (Roche), pep-statin A, E-64, PMSF) was added. Approximately 1 ml was eluted into an eppendorf tube. Elution was repeated 2 x with 1 mL of CEB. Samples were taken at all the individual steps of the purification procedure. Samples were either taken directly or were TCA-precipitated prior to analysis via SDS-PAGE and Western blotting. When indicated, protein samples were further concentrated via spin-columns (Roti-Spin, Roth) prior to SDS-PAGE. Protein bands were cut out of the gel and sent to mass spectrometric analysis.

### Example 14: Mass spectrometry

Mass spectrometry of proteins was performed by Dr. Michael Kiess (Toplab GmbH, Germany). Proteins, submitted as gel slices were reduced with DTT and alkylated with IAA (Iodacetamide) and digested with trypsin at 37° C overnight. The obtained peptides were analyzed by MALDI-TOF-MS (4800 Proteomics Analyzer, Applied Biosystems), positive ionization mode (negative mode, when indicated). Peptide masses were analyzed with PeptideMap software. Further analysis was done with Mascot, a search engine to identify proteins from primary sequence databases (Perkins et al., 1999).

### Example 15: SDS-PAGE and Western blotting

SDS-PAGE was performed as already described in the VKORC1-section. For positive controls, purified human prothrombin was purchased from ERL (Enzyme Research Laboratories). PAA-gels were either stained with Coomassie Brilliant Blue R-250 (Neuhoff et al., 1985) or colloidal Coomassie blue (Gel Code Blue, Pierce) according to manufactorers' instructions. Silver staining was performed according to (Shevchenko et al., 1996). Pro-tein bands were cut out and submitted for mass spectrometry (Toplab GmbH, Germany) Western Blots were performed according to Towbin et al. (1979) on a Mini Trans-Blot Sys-tem (Biorad). Proteins were either blotted either onto nitrocellulose (Amersham Hybond ECL) membrane or PVDF membranes (Biorad). For detection of prothrombin rabbit polyclonal anti-prothrombin (Hyphen) and sheep polyclonal anti-prothrombin conjugated with HRP (Enzyme Research Laboratories) were purchased from Coachrome. Murine anti-gamma carboxyglutamyl antibody was obtained from American Diagnostics. Furthermore, anti-calmodulin binding peptide antibody (Millipore), anti-actin antibody (MP Biomedicals), anti S-tag antibody (Abcam) and anti GGCX antibody (Abcam) were used in these studies. Secondary Antibodies, conjugated with HRP (horseradish peroxidase) were obtained from Jackson Immunoresearch.

### Example 16: Immunoprecipitation of Prothrombin

Preparation of Protein A beads: Protein A sepharose (Sigma) 250 mg was swollen in Phosphate buffered saline, PBS (pH 7.4, 0.1 mol/l, 0.9 % NaCl). The supernatant was discarded. 6 ml of PBS (10 mM, 0,9 % NaCl, 1 % BSA) was added and incubated for 1 h. This step was repeated. Finally the sepharose was washed 3 x with PBS and stored in presence of 0,02 % NaN₃. Extraction and wash Buffers for IP: 50 mmol/L TRIS/HCl pH 8, 150 mmol/L NaCl, 1 mmol/L EDTA, 0,02 % (v/v) Trition X-100, 1/100 PMSF (1 mmol/L), 1 % (v/v) Fungal protease inhibitor cocktail (Sigma). Wash buffer and extraction buffer composition are basically the same, but washing buffer contained only 0.3 mmol/L PMSF and 0.3 % (v/v) fungal protease inhibitor cocktail. Immunopreciptiation: Mycelium was harvested and frozen with liquid N2. Mycelium was homogenized by grinding in liquid N2. Eppendorf tube was filled approximately to its 500 l level. 2ml tubes were filled with grinded mycelial powder (liquid N2) and 1 ml of freshly prepared extraction buffer was added. Samples were mixed by vortexing and incubated on a rotor for 20 min at 4 °C. Clearance of samples was achieved by centrifugation for 2 x 13.000 rpm at 4 °C, the pellet was discarded. The cleared supernatant was used for determination of the protein concentration, BCA assay (Pierce). Protein concentration of the input was set to approximately 3,5 mg/ml. 800 µl of the cell extracts were precipitated with 12,5 - 15 µl prothrombin-specific antibody (1 mg/ml, Hyphen) on rotor at 4°C overnight. The Gla specific precipitation was achieved with 1,8 ml cell extracts and 10 µl anti-gla antibody (1 mg/ml, American Diagnosics). After 12 hours, 75 to 100 µl protein A slurry (=50 µl swollen beads) was added and incubated for 4 h on a rotor at 4 °C. (Protein A slurry was washed 3 times before adding it to protein extracts.) The precipitates were centrifuged for 1 min at 500 x rpm and washed 3 x 800 µl freshly prepared extraction buffer. 50 µl of Laemmli Buffer containing DTT was added to the samples and heated to 95°C for 5 minutes. (Laemmli, 1970) Western transfer was done in modified Towbin Buffer (48 mM Tris, 39 mM glycine, 20 % (v/v) methanol, pH 9.2, 0.025 % (w/v) SDS for 80 min.

### Example 17: Vitamin K uptake in Aspergillus nidulans

The vitamin uptake ability of *A.nidulans* was studied with growth tests in Petri dishes. In addition, vit.K uptake and unspecific resorption of vit.K to the fungal cell wall was analyzed in liquid cultures. According to growth tests in Petri dishes, 752 µg of vitamin K was quantified in 100 mg DW (dry weight) of lyophilized fungal mycelium. However, due to the set-up of this experiment it remains unclear if the vitamin K is either attached to the cell wall or actively taken up into the fungal cell. To clarify this, a second growth test comparing viable and heat-inactivated mycelium was performed. The results are shown in Table 1 and clearly demonstrate that vitamin K is only to a minor extent unspecifically associated with the fungal cell wall, indicated by the values obtained from inactivated cultures. The maximal amount of vit.K unspecifically attached to the cell wall is approximately 20 µg per 100 mg (dry weight) of fungal mycelium. The majority of vitamin K was found in viable cells. The viable mycelium was able to take up approximately 8% of the vitamin K supplied through the culture medium (Hill et al.), more precisely 1,14 mg / 100 mg mycelium from the 50 mg vit.K supplied in total (Table 2).

**Table 2: Vitamin K1 updake ability of Aspergillus nidulans strain YR 23.5. Vitamin K was added to liquid cultures, the final concentration as indicated. Fungal mycelium was inactivated by boiling in a microwave oven. Values which were obtained with inactivated fungal cell mass indicate the unspecific association of vitamin K to the fungal mycelium.**

| **Fungal cell mass** | **\|Vitamin K added** | **\|Absolute quantities (vitamin K** / **DW)** | **\|Uptake (vitamin K** / **DW)** |
|---|---|---|---|
| active | 500 µg/ml | 1140,0 µg/100 mg | 1122,1 µg/100 mg |
| inactive | 500 µg/ml | 17,9 µg/100 mg | - |
| active | 50 µg/ml | 183,0 µg/100 mg | 169,1 µg/100 mg |
| inactive | 50 µg/ml | 13,9 µg/100 mg | - |
| DW = Fungal mycelium (dry weight) | | | |

### Example 18: VKORC1 and GGCX targeting strategies in A.nidulans

VKORC1 and GGCX are ER membrane residing proteins (Tie et al., 2005). Unfortunately, there is no reliable method to predict the localization of a mammalian ER membrane protein in filamentous fungi. In contrast, the localization of endogenous (membrane) proteins in the secretory pathway of yeast and filamentous fungi is well studied (Choi et al., 2003). VKORC1 and GGCX expression strategies focused on membrane targeting. To achieve this, VKORC1 and GGCX were fused to a fungal cellular ER-targeting sequence MnsA. MnsA is the A. niger homologue of yeast Mns1 (alpha-1,2-mannosidase). This ER-leader sequence was identified in a combinatorial genetic library approach, initially developed for the targeting of ER residing enzymes involved in protein glycosylation (Kainz et al., 2008; Choi et al., 2003). As the localization of the chimeric VKORC1 proteins was planned to be studied by confocal fluorescence microscopy, fluorescence tags were linked to the GGCX and VKORC1 constructs. For ER controls, GFP moieties were attached to different MnsA leader sequences varying in their luminal stem region length. For negative (cytosolic) controls fluorescence proteins without any fungal targeting sequence were generated. Strong and inducible fungal promoters, the alcA(p) (VKORC1) and the EREuraRS (p) (GGCX) were chosen to control transcription (Pachlinger et al., 2005). Total protein extracts of A.nidulans strains, transformed with the individual control construct, were prepared and TCA precipitated. Immunoblots of GFP expressed under ERE-uraRS promoter are shown in Figure 1. GFP expression can be induced upon addition of DES. GFP migrates according to its molecular size of 27 kDa on SDS-PAGE. A basal level of expression can be observed, indicating a leakyness of the ERE promoter or a contamination caused by estrogenic substances in the preceding liquid culture (Figure 1 A). MnsA2:GFP was detected on immunoblots according to the calculated size of 42 kDa (Figure 1 B). Next, chimeric VKORC1 and GGCX constructs were transformed in *A.nidulans* YR23.5 strain. Stable integration into the fungal genome was verified by PCR. Transcriptional levels were analyzed by quantitative PCR. Considerable variations of relative mRNA values were observed and are most probably due to multiple integrations and locus effects. Transformants, showing highest relative chimeric VKORC1 or GGCX transcript levels were chosen for further analysis of protein expression by Western blotting. Interestingly, none of these constructs was detected on immunoblots. As transcriptional analysis of strains transformed with VKORC1 and GGCX chimera showed acceptable specific mRNA levels, poor translation due to unfavourable codon usage of the human VKORC1 or GGCX sequence might be one explanation for difficulties in protein expression. Though, the nucleotide sequences of VKORC1 and GGCX were codon-optimized for improvement of translation in *A.nidulans.* In the adapted targeting approach it was decided to replace the predicted N-terminal membrane domain of VKORC1 by a fungal ER-targeting membrane domain. To facilitate a straightforward
cloning strategy a V29M mutation in the aa sequence of VKORC1 was introduced. Besides fluorescence microsopy, a biochemical approach by fungal cell fractionation was chosen. Therefore further VKORC1 and GGCX constructs were cloned in which the GFP-tag was replaced by an S-tag. The designed VKORC1 and GGCX constructs were transformed in *A.nidulans* YR 23.5. The stable integration into the chromosomal DNA of *A.nidulans* was analyzed by Southern blotting. As expected for the majority of the strains transformed with VKORC1^{V29M}: :Stag or GGCX::Stag constructs, multiple integrations into the genome were observed. Quantitative PCR data showed that EMK induction of VKORC1^{V29M}: :Stag under the control of alcA promoter resulted in 500 up to 1500 fold up-regulation of VKORC1^{V29M}: :Stag (VmS) transcriptional levels. The transcription level of VKORC1 constructs with fungal ER leader sequences was considerably lower compared to AN-VmS strains. Moreover, total protein extraction of strains harbouring codon-optimized VKORC1^{V29M}: :Stag or VKORC1^{V29M}construct showed a very strong and stable expression profile (Figure 1). VKORC1^{V29M}: :Stag was detected according to the calculated mass at 19 kDa on the membrane. In contrast, strains harbouring VKORC1^{V29M} (variants) in addition to a fungal leader sequence showed lower expression levels and less stable, partially degraded protein. For an unambiguous identification of fungal expressed VKORC1, an antibody specific for human VKORC1 was generated. To our knowledge, there is no reliable anti-human VKORC1 antibody commercially available. The custom-made VKORC1 antibody, which was designed and purified in our laboratory can be used for reliable detection of fungal expressed VKORC1, even in total cell lysates (Figure 1). Northern analysis of GGCX transformants revealed high transcription levels of native GGCX:Stag sequence. In contrast, measurable mRNA levels of constructs harboring an fungal ER leader were not detected. Protein expression levels of AN-GS induced with variable concentrations of DES were analyzed. Induction of EREuraRS promoter at concentration of 1 nmol/L led to highest expression levels of GGCX::Stag. GGCX:Stag is detected according to the calculated mass, ca. 90 kDa on the membrane. Non-induced samples did not show detectable levels of GGCX::Stag (Figure 1).

### Example 18a: Analysis of GGCX and VKORC1 membrane localization

The expression of GFP without targeting sequence resulted in a cytosolic distribution within the fungal cell (Figure 1). A specific localization pattern for MnsA2::GFP could be observed by fluorescence microscopy (Figure 2). Comparison of a DAPI stained fungal nuclei was performed and assigned to the localization pattern generated by MnsA2::GFP. The proximity and the apparent co-appearance of MnsA2::GFP with DAPI stained nuclei indicate a proper ER membrane localization. Moreover, the GFP fluo-rescence of the strain harbouring the MnsA2::GFP construct is mostly overlaps with the fluorescence signal obtained by an ER-membrane specific dye (ER-tracker) (Figure 2). To further investigate localization of MnsA::GFP a biochemical approach by fungal cell fractionation was chosen. The co-purification of VKORC1 constructs with the microsomal fraction (including Endoplasmic Reticulum) was analyzed by Western blots with specific antibodies. First, microsomal prepartions AN-M2-GFP strains (construct: mnsA2::GFP) was performed and the fractions were analyzed by Western blotting (Fig-ure 7.2). A flow chart, wich presents the whole cell fractionation procedure and indicates which samples were further analyzed, is shown in Figure 2. The figure was taken and modified after (Abas and Luschnig, 2010). The individual fractions obtained for strain AN-M2-GFP were generated by step-wise preparation of microsomes and are shown in Figure 2. Results clearly demonstrate an enrichment of MnsA2::GFP in the microsomal fraction and absence of the ER-membrane targeting protein in the corresponding soluble supernatant (Figure 2). Compared to the input, the relative quantity of MnsA::GFP was significantly increased in the microsomal fraction. Next, the method was applied for strains expressing various VKORC1^{V29M}constructs. VKORC1^{V29M}: :Stag expressed without fungal targeting peptide showed co-purification with the microsomal membrane fraction (Figure 3). Western blots probed with S-tag antibody clearly demonstrated a substantial increase of VKORC^{129M}: :Stag quantity in the microsomal fraction compared to quantities in crude cell homogenates (Figure 3). In contrast, VKORC^{129M}: :Stag was not present in the soluble supernatant fraction, which was obtained after the 120,000 x g centrifugation step. The putative ER-membrane localization of VKORC1 is supported by identical signal patterns obtained by fluorescence microscopy of MnsA2::GFP and VKORC1**::**GFP (Figure 3 and 2). Likewise, GGCX:Stag was clearly detected in the microsomal fraction and was strongly enriched compared to the input. For samples obtained from cultures without DES addition, small quantities of GGCX:Stag were detected in the microsomal fraction on lane 5 (Figure 3). This is consistent with data of previous Western blot and Northern blot analyses. Our data clearly support the membrane localization of GGCX::Stag as the protein could be enriched in the microsomal fraction and failed to be analyzed in the soluble fraction after the final 120,000g centrifugation step. Furthermore, the microsomal preparation and fluorescence microscopy data of control construct AN-M2-GFP and GGCX::Stag showed comparable localization patterns (Figure 3).

### Example 19: Combining VKORC1 and GGCX activities in one strain

The combination of VKORC1 and GGCX activites in one strain was either achieved by crossing AN-VmS with AN-GS or by transformation of strain AN-VmS with the hER and GGCX:Stag construct. Transformants were further screened by PCR and Northern blotting for expression of both constructs. Induction of VKORC1 V29M : :Stag by EMK and induction of GGCX::Stag by DES was clearly observed. Total protein lysates of several AN-VmS-GS transformants were TCA-precipitated and analyzed by immunoblotting. Parallel detection of GGCX:Stag and VKORC1:Stag was feasible with an S-tag antibody. Western blots of four different transformants, where protein samples were previously separated on either a 10 % or a 15 % SDS-PAA gel are shown in Figure 4. Parallel detection is demonstrated on the 15 % gel, where VKORC1 V29M::Stag migrates according to its calculated mass of 19 kDa and GGCX::Stag is detected in the high mass fraction of the blot. On the blot of the 10 % gel GS can be detected according to its calculated mass of ca. 90 kDa. Consistent with previous results, VKORC1 expression seems to be tightly controlled by the alcA promoter, whereas GGCX expression, controlled by ERE promoter shows some background expression under non-induced conditions. Various transformants and biological repetitions of the experiment demonstrated the independent co-expression of both VKORC1 and GGCX in one strain (Figure 4). As VKORC1 and GGCX constructs were already shown to co-purify with the membrane fraction (Figure 3) the set-up of a GGCX and VKORC1 activity assay was started. However, co-localization of VKORC1: :mcherry and GGCX::GFP was verified by fluorescence microscopy (Figure 3 B).

### Example 20: VKORC1 in vitro activity assay

In classical VKORC1 assays (Wallin and Martin, 1985) microsomes are incubated with vitamin K Epoxide (K>O) and DTT is added as in vitro reductant to start the reaction. Different vitamin K forms, K>0 and K are extracted and analyzed by reverse phase HPLC and quantified UV detection (Wallin and Martin, 1985). The low sensitivity of the UV detector was found unsuitable for analyzing generated K levels, as activity of VKORC1 in fungi was not yet demonstrated (personal communication: L. Gille). Consequently, an alternative electrochemical detection was used to quantify K and the enzymatic activities were calculated as K (ECD) / KO (UV) ratios. Thus random errors, resulting from inaccuracies in pipetting could be eliminated. In preliminary experiments fungal cell extracts did not seem to negatively interfere with the assay, as neither unspecific KO reduction nor degradation was observed. As soon as it was observed that a fungal ER targeting leader seemed not to be necessary for proper localization, VKORC1 in vitro activity assays were performed with microsomes of strain AN-VmS. Conditions for VKORC1 assay were optimized by standardizing the cell disruption method, buffer composition and incubation time. In vitro assays with crude extracts were performed to clarify the influence of a fungal background (fungal proteins, possible fungal related inhibitors) on VKORC1 activity. AN-RFP control strains (mRFP1 expression) (Figure 5 A1), sharing an identical genetic background to AN-VmS strains, were expected to be suitable as a negative control for the assay. Incubation of vit KO in the assay buffer was used to analyze unspecific KO reduction by DTT. Significant K / K>O ratios confirmed the VKORC1 and DTT dependent K>O reduction ac-tivity in engineered *Aspergillus nidulans* crude cell extracts. Negative controls with sam-ple buffer showed that even under long-term incubation conditions and DTT addition, that only negliable amounts of KO were reduced. K / K>O ratios of AN-RFP strain remained in the same order of magnitude of ratios as those obtained for the sample buffer (data not shown). Next, VKORC1 constructs containing the corrected V29M mutation to VKORC1 wildtype amino acid sequence were designed (Figure 5 A2). A VKORC1 construct lacking the S-tag to avoid a putative negative interference of the tag on VKORC1 activity was cloned and transformed in A.nidulans (Figure 5). Total cell extracts of all VKORC1 expressing strains showed high K / K>O ratios de-pendent on DTT addition (Figure 5 B1). K / K>O ratios of AN-V strains (VKORC1 construct) were at least 1.5 fold increased compared to ratios of strains harbouring the VKORC1 V29M::Stag construct. The assay buffers and incubation of a negative control strain expressing mRFP1 showed no Vit.K1-Epoxide reduction abil-ity (Figure 5 B1). Postmitochondrial supernatants were generated by clearing crude cell extracts by a centrifugation step of ca. 5000 x g. In theory, the postmitochonridal (PM)-supernatant includes microsomes, membrane fragments, ribosomes, while the pellet contains cell debris, nuclei and mitochondria. PM-supernatants were used to perform VKORC1-assays for monitoring effects of cell debris, mitochondria etc. on VKORC1 activ-ity. A potential loss of microsomes, accompanied by decrease in VKORC1 activity due to cell disruption and centrifugation step was expected; consequently a scale up the reaction volume and a decrease of the Vit.K epoxide concentration in the assay (see Materials and Methods) was performed. The decreased substrate (Vit.K1 epoxide) concentration in association with a scaled up reaction volume, clearly demonstrate VKORC1 activity in AN-VPM supernatants (Figure 5 B2). PM supernatants of a negative control strain expressing mRFP1 showed no K>O reduction.

### Example 21: Prothrombin, a VKD gamma-carboxylation reporter

A gamma-carboxylation reporter is a useful tool for directly demonstrating GGCX activity in vivo, as gamma-carboxylase activity was not clearly shown in GGCX in vitro assays. Reporter constructs were based on prothrombin, a well studied VKD dependent blood clotting factor (for reviews see Hansson and Stenflo (2005); Suttie (2009)). The design of reporter constructs was based on the same principles as already outlined for VKORC1 and GGCX. Hence, prothrombin cDNA was codon-opitmized for expression in A.nidulans. In our study four different constructs were cloned and transformed (Figure 6 A1). Either the full length prothrombin or the prothrombin gla domain were fused with pre and pro pep-tide(s) of A.niger glucoamylase. Furthermore a TAP (tandem affinity purification) tag was added to the C-terminus of the reporter constructs (Figure 6 A1). The plasmids were transformed in AN-VmS-GS strain. The full length prothrombin (PT) or the gla-domain (GD), comprising the native prepro sequence showed a stable and strong protein expression upon EMK induction (Figure 6 A2). Moreover, the constructs with prepro (GD2) and pre (GD1) leader sequences of Glaa were analyzed for their expression. Replacement of the prothrombin signal peptide by a fungal signal peptide (pro) or prepro sequence did neither effect protein expression level, nor protein stability (Figure 6 A1). Interestingly, constructs harbouring the Glaa prepro site as leader sequence showed two bands on the Western blot. The Glaa pro site carries a Kex2 cleavage site and it can be assumed that these constructs are already processed by fungal Kex2 like proteases. As prothrombin expression was working reliably and no data concerning the influence the Glaa propeptide or signal peptide on translocation or secretion were available at that time, we focused on expression and purification of full length prothrombin::TAPtag (PT). Initial purification studies were performed with total cell extracts of AN-VmS-GS-PT, as the strains with optimized VKORC1 were not available at that time. PT was found in high amounts in the soluble fraction of total cell lysates, which were taken for purification. Performing the full purification protocol resulted in high purity of prothrombin::TAP but only low yields were achieved. Consequently, prothrombin purifications were carried out with an adapted and up-scaled one-step protocol (IgG-column) to achieve protein quantities suitable for posttranslational modifications (PTM) - analysis. The up-scaled protocol enabled us to achieve an approximately 10-fold increment of PT concentration in the gel, where spots were cut and sent to MS-analysis (Figure 6 B1). The mass spectrum (PMF, peptide mass fingerprint) was analyzed with MASCOT and, as expected, prothrombin was unambiguously identified (Perkins et al., 1999)(Figure 6 B2). Peptide 44-52 and peptide 59-86 represent the gla-domain of the trypsin digested prothrombin. Peptide 44-52 was identified by MS, but only with unmodified glu residues. MS analysis failed to detect peptide 59-86 either in an unmodified or in a modified (= gamma-carboxylation of Glu) form (Figure 6 B2).

Thus, an additional strategy was pursued to identify putative gamma-carboxyglutamyl residues in fungal generated prothrombin::TAP. A monoclonal antibody against human gamma-carboxyglutamyl residues (Gla) was evaluated in the next step (Brown et al., 2000). First experiments were carried out with fully purified (IgG + Calmodulin column) or partially purified prothrombin::TAPtag (IgG column column). Purified prothrombin was either expressed under GGCX induced (+DES) or non-induced conditions (-DES). As already discussed above, significant protein amounts of GGCX were detected under GGCX non-induced conditions. For this reason the use of prothrombin::TAP (-DES conditions) as mock control may complicate interpretation of results. Prothrombin constructs without any artificial tag seemed to be essential for evaluation of the gla-specific antibody (mentioned above). Therefore, a new prothrombin construct without affinity tag, named prothrombin (P) was designed, cloned and transformed in strain AN-V (strain with optimized VKORC1 activity) (Figure 7). High protein expression levels could be observed dependent on EMK induction for prothrombin and prothrombin::TAPtag (Figure 7). Western blots clearly showed that prothrombin migrates according to its calculated mass of 70 kDa, whereas prothrombin::TAP migrates at higher mass (Figure 7). The strain AN-V-P was transformed with the hER and GGCX::Stag construct and was further characterized for its in vivo gamma-carboxylation activity. Cultures were pre-grown over night in standard minimal medium with carbonate / phosphate buffer system (Hill et al.). Pre-grown biomass was induced with EMK and DES (VKORC1, prothrombin and GGCX::Stag induction) and vitamin K1 (Konakion) was added. Western blots of TCA precipitated crude extracts were performed and prothrombin was clearly detected. Unfortunately, development of blots with gla specific antibody did not result in a distinct identification of potentially gamma-carboxylated glu residues. Even extended exposure time or the use of a maximum sensitivity ECL substrate did not lead to a clear picture.

Hence, prothrombin enrichment and a partial purification was achieved via immunoprecipitation. The precipitated prothrombin was then analyzed for PTM by Western blotting with Gla-specific antibody. The already established prothrombin antibody (HRP-conjugated, ERL) is not appropriate for IP (immunoprecipitation), consequently we evaluated a rabbit polyclonal prothrombin antibody (Hyphen). The antibody seemed to work reliably, immunoblots of TCA-precipitated total cell lysates showed high specificity in a fungal protein background. For IP, total cell lysates of strains AN-V-GS-P and AN-V-P were prepared and prothrombin antibody was added for precipitation. Protein A sepharose was used to bind the antibody-prothrombin-precipitate from the soluble fraction of total cellular protein. Next, the protein A slurry was washed several times and finally eluted in Laemmli buffer. Western blots, hybridized with prothrombin-HRP(ERL) antibody, showed equal precipitation and/or equal loading on the blot, independent on GGCX expression or Vitamin K addition (Figure 8 A). The blots were subsequently probed with the anti-GLA antibody. Strikingly, prothrombin could only be detected if Vitamin K was added and GGCX was co-expressed ((Figure 8 A)). In an experimental swap, a pro-thrombin IP using the anti-GLA antibody was performed. Again, only if Vitamin K was added and GGCX expressed, prothrombin precipition with the anti-GLA antibody was ob-served (Figure 8 B). Another IP was performed and an additional negative control, strain AN-Vms-GS, which is deficient in prothrombin expression, was included. Total cell lysates of strains AN-V-GS-P, AN-V-P and AN-VmS-GS were prepared and prothrombin antibody was added for precipitation. Western blots, which were developed with prothrombin-HRP(ERL) antibody, showed equal prothrombin precipitation for strains AN-V-GS-P and AN-V-GS (Figure 8 C). As expected, no signal was observed for the prothrombin IP of cell lysates for strain AN-VmS. The blots were subsequently probed with the anti-GLA antibody and again prothrombin could only be detected if vitamin K was added to the cultures and GGCX was co-expressed (Figure 8 C).

### Example 22: Discussion and Summary

The minimal requirements for a functional heterologous VKD protein carboxylation system strongly rely on functional expression of VKORC1, GGCX and a supply of vitamin K hydroquinone. Vitamin K is not required for fungal life and it is commonly known that fungi have the widespread ability to grow on a wide variety of carbon sources. Fungi are involved in different modes of hydrocarbon metabolism (Prenafeta-Boldu et al., 2006). Moreover, several *Aspergillus* species were shown to be capable of growth on phenol or styrene as sole carbon and energy source (Jones et al., 1995). Being aware of fungal metabolism vitamin K uptake and metabolism of *Aspergillus nidulans* strain YR23.5 was investigated. Growth tests on solid medium and submerged cultures were performed and showed that vitamin K remained stable in the fungal cell, at least under standardized growth conditions, providing sufficient alternative nutrients. Results further indicate that adequate intracellular vitamin K levels can be provided, which are required for in vivo gamma-carboxylation reactions. Several approaches for functional expression of VKORC1 and GGCX in *A. nidulans* were undertaken. VKD proteins proceed from the ER through the secretory pathway, being sequentially exposed to different catalytic activities. Several processing steps rely on previous enzymatic reactions (Berkner, 2008). While proper localization is important, additional factors in the environment, such as pH, may influence the enzymatic activities (Choi et al., 2003; Gerngross, 2004).

In the engineering of fungi to mimick human type N-glycosylation a protein targeting approach was successfully used. Several yeast and fungi species were engineered for humanization of N-glycosylation. A breakthrough was achieved by Glycofi Incorporation (US). Glycofi researchers engineered the yeast *Pichia pastoris* to secrete human glycoproteins with fully complex terminally sialylated N-glycans (Hamilton et al., 2006). In this study, combinatorial leader peptides were fused to catalytic domains and chimeric enzymes with optimal activity in the appropriate compartment were identified. In a previous study MnsA (*A.niger* alpha 1,2-mannosidase) was identified as putative leader peptide for ER-membrane targeting of proteins (Kainz, 2006; Kainz et al.,2008). Several constructs comprising MnsA2 as targeting leader were designed. MnsA2 is a truncated form of MnsA, deficient of the catalytic domain, and with a defined length of the stem region that is fused to the N-terminus of VKORC1. The inducible promoter alcA(p) was taken for VKORC1/mRFP1 based constructs whereas EREuraRS promoter was used to control GGCX/GFP based constructs. The choice of promoters should assure independent regulation of each enzyme. However, we observed, that both promoters are not necessarily tightly regulated (Figure 1 and 4). All GFP, mRFP or MnsA based control constructs showed reliable expression upon induction. Furthermore a specific localization, presumably ER, was observed for MnsA2::GFP protein by fluorescence microscopy. In contrast, none of the VKORC1 or GGCX chimera was detected by Western blotting. As transcription analysis revealed strongly inducible levels of VKORC1 and GGCX, we concluded a poor codon usage of the human cDNA. Impressive improvement in expression of nonfungal heterologous proteins using codon-optimized synthetic genes was already demonstrated (Koda et al., 2005), so the VKORC1 and GGCX sequences were synthesized in vitro in a codon-optimized form with increased GC levels for optimal expression in *A.nidulans.* A valine to methione mutation was introduced in the sequence for cloning purposes. V29L mutation in human VKORC1 was demonstrated to leave VKORC1 activity almost unaffected, while leading to warfarin resistance (Rost et al., 2004). Although codon optimization of constructs led to a detection of mnsA2/VKORC1 or GGCX chimera, a stable reliable expression was never observed. When designing ER targeting constructs the three transmembrane model of VKORC1 was taken into account (Tie et al., 2005). The model suggests that the N-terminus of VKORC1 resides in the ER lumen. The fused MnsA leader peptide with variable length of the luminal stem region would consequently not interfere with native VKORC1 membrane topology. Recently, the crystal structure of a bacterial VKORC1 homolog became available and the topology model, which was proposed by Tie was challenged (Li et al., 2010). The model suggests four transmembrane domains with the VKORC1 N-terminus in the cytosol. This topology is incompatible with the applied MnsA leader targeting strategy, as MnsA has its C-terminal stem region in the ER-lumen. Hence, poor expression and partial degradation of MnsA-VKORC1 chimera might be caused by negative interference of MnsA on VKORC1 topology. In a straightforward approach we evaluated if the native GGCX and VKORC1 sequence is sufficient to achieve ER-(membrane) localization in *Aspergillus.* Wang et al. (2005) proposed the di-lysine motif (KXKX) at aa 159-163 in VKORC1 sequence as ER retention signal. The carboxyterminal di-lysine motif is suggested to be conserved across eukaryotes (Teasdale and Jackson, 1996). In contrast, Rost et al. (2004) showed that VKORC1 tagged with GFP on its C-terminus is localized in the ER-membrane. Consequently, it is plausible that another retention signal besides the di-lysine motif is involved in the ER retrival of VKORC1, probably somewhere in the transmembrane domain (Sato et al., 2003). We could demonstrate stable expression of codon optimized GGCX::Stag and VKORC1^{V29M}: :Stag. Membrane localization of GGCX and VKORC1 was analyzed with different techniques.
I) Microsomal preparation of cell lysates of strains expressing either GGCX::Stag or VKORC^{129M}: :Stag were performed. Both enzymes were strongly enriched in the microsomal fraction. A comparable enrichment in microsomal fractionation was shown for positive control MnsA::GFP. Co-purification of GGCX::Stag with the microsomal fraction is a strong indication for proper membrane localization, however it remains ambiguous if GGCX::Stag and VKORC^{129M}: :Stag reside in the ER-membrane.
II) Native GGCX or VKORC1 sequence was tagged with GFP or mCherry. GFP fluorescence of GGCX::GFP and VKORC1**::**GFP was observed as a tubular network. Comparable fluorescence images were obtained for the strain harbouring MnsA2::GFP construct. As MnsA2 is a native fungal ER-residing protein, we suggest ER-membrane localization for VKORC1 and GGCX, when expressed in A.nidulans. Strikingly, co-expression of VKORC1: :mCherry and GGCX::GFP led to a co-localization of both enzymes. However, we cannot exclude additional tER and/or early Golgi localization. Further studies, including several controls, can determine both VKORC1 and GGCX localization in the fungal cell. Fungi have a versatile, flexible metabolism and are capable of partial transformation of hydrocarbons (Prenafeta-Boldu et al., 2006). The versatile fungal metabolism could interfere with an assay based on quantitative Vitamin K1 analysis, especially if long incubation times are necessary and low enzyme activities are expected. In preliminary experiments fungal cell extracts did not seem to negatively interfere with the assay, as neither unspecific KO reduction nor degradation was observed. Adaptation of the VKORC1 in vitro assay was performed stepwise and we observed that the most critical point for assaying VKORC1 in crude cell extract is the disruption of the cells. However, we were able to show DTT dependent VKORC1 activity in A.nidulans total cell lysates or even more clearly in postmitochondrial supernatants. Reduction of vit. K1 quinone can be performed either by VKORC1, VKORC1L1 or NADPH-dependent quinone reductases (Westhofen et al., 2011). In contrast, KO reduction is specific for VKORC1 or to a limited extent for VKORC1L1. From, in vitro assays we concluded that VKORC1 is functionally expressed and membrane localized. However, kinetic analysis of fungal expressed VKORC1 was not performed, so we cannot draw further conclusions. A gamma-carboxylation reporter is a helpful tool for directly demonstrating GGCX activity in vivo, as gamma-carboxylase activity was not assayed in vitro. In order to not overburden the translocation, translation and secretion machinery of the fungus by overexpressing four mammalian proteins simultaneously we decided to control the expression of reporter constructs by the alcA promoter. It was assumed that expression of two heterologous genes under alcA promoter limits expression levels but prevents negative effects of over-expression on physiology of the fungus (Gwynne et al., 1989; Felenbok, 1991). The fusion of a heterologous gene with a fungal gene encoding a well-secreted protein is a state of the art method to increase heterologous secretory protein yields in Aspergillus (Gouka et al., 1997). However, if prothrombin constructs would be expressed using the fusion carrier strategy (Gouka et al., 1997), the accessibility of the CRS (carboxylase recognition site) might be strongly impaired. Moreover, the N-terminal position of the propeptide is highly conserved among VKD proteins. In contrast, secretion is much less efficient, when only the short amino acid sequence of the signal peptide and the propeptide (secretion signal) of fungal glucoamylase (Gouka et al., 1997; Broekhuijsen et al., 1993) are fused to a heterologous protein of interest. As the main goal of this study was to achieve proof of concept, the putative drawback of reporter protein secretion without a fungal carrier was neglected but is feasible to achieve at any further point of process optimization. Either the full length prothrombin or the prothrombin Gla domain were fused with pre and pro peptide(s) of A.niger glucoamylase. Replacement of the prothrombin signal peptide by a fungal signal peptide (pro) or prepro sequence did neither effect protein expression level, nor protein stability. Full length intracellular prothrombin expression was working reliably and was taken for analyis of PTM gamma-carboxylation by mass spectrometry. Performing the full purification protocol resulted in high purity of prothrombin::TAP but only low yields were achieved. Therefore, prothrombin purifications with an adapted and up-scaled one-step protocol (IgG-column) was performed to achieve protein quantities suitable for PTM-analysis. MS analysis demonstrated that the sequence coverage was dramatically increased, using higher proteins quantities. Peptide 44-52 was identified by MS, but only with unmodified glu residues. MS analysis failed to detect peptide 59-86 either in an unmodified or in a modified (= gamma-carboxylation of Glu) form. It was assumed, if gamma-carboxylation would be functional in an engineered A.nidulans strain, some prothrombin molecules carry a mixture of carboxylated and non-carboxylated glutamate residues and some prothrombin molecules may not carry any gamma-carboxyglutamate residues in the Gla-domain. Moreover, gla is difficult to analyze by mass spectrometry due to the properties of this negatively-charged residue (Hallgren et al., 2012; Kelleher et al., 1999).

In addition, many factors like fungal in vivo VKORC1 activity and GGCX activity, proper translocation of PT, vitamin K distribution within the fungal cell and consequences of parallel overexpression of four mammalian proteins on physiology and fitness of the strain may negatively interfere with the process. However, it cannot be excluded that each of these points may severely affect or abolish the heterologous gamma-carboxylation machinery in A.nidulans. Consequently, a more sensitive approach to analyze putative gla's in fungal expressed pro-thrombin was needed. Monoclonal antibodies that specifically recognize gla residues in proteins and peptides were produced and characterized by Brown and coworkers (Brown et al., 2000). Decarboxylation or the replacement of gla with glutamate destroys the epitope, whereas binding of the antibody is maintained when the sequence surrounding the gla residue is altered. The protein A moiety of the TAP-tag led to false positive results when analyzing gamma-carboxylation with several antibody species. Furthermore, the leakiness of EREuraRS promoter, which controls GGCX expression complicates interpretation of results. We co-expressed prothrombin with its native prepro sequence in a strain with VKORC1 and GGCX or in a strain with VKORC1 only. As Western blots performed with total cell extracts did not lead to a clear result, in further experiments prothrombin was enriched via immunoprecipitation. Lower prothrombin quantities were found in cell extracts obtained from cultures grown in presence of vitamin K and consequently less prothrombin was immunoprecipitated. It seemed that addition of vitamin K has a negative not characterized influence on prothrombin expression. However, prothrombin is expressed under the control of A.nidulans alcA promoter and the strength of this promoter depends on many different parameters, i.e. a complete repression by high concentrations of a preferred carbon source such as 1% glucose(Nikolaev et al., 2002; Felenbok, 1991). For this reason 0,2 % (w/v) fructose in media was used, which is a good compromise between biomass generation and protein (prothrombin) expression. However, we observed that the carbon status in the cultures and consequently the prothrombin expression is batch dependent and not negatively influenced by vit. K addition. Strikingly, Prothrombin was exclusively detected by the gla antibody in samples where Vitamin K was added to the culture and GGCX and VKORC1 were co-expressed. On the resulting Western blots of the prothrombin IP a dominant band at 55 kDa was observed (see Figure 8). This band most probably results from protein A, which was used for IP. For cyanbromide activated sepharose it is known that a small but constant leakage of the coupled ligand, protein A occurs (Sigma Aldrich Data sheet). Furthermore this distinctive, dominant band can only be detected upon hybridization with a mouse antibody (anti-GLA), while hybridization with a sheep antibody (prothrombin-HRP) did not result in a signal. In a biological repetition another negative control was provided by strain AN-VmS-GS (expression of VKORC1^{V29M}GGCX: :Stag). As expected no prothrombin was precipitated, but the 55 kDa band (most probably protein A) was detected upon incubation with the gla specific antibody. In an antibody swap experiment the anti-gla antibody was used in the IP step. This experiments clearly showed that only if GGCX, VKORC1 are co-expressed and vit. K1 is added to cultures prothrombin can be precipitated. Taking together all biologically independent experiments, we have a strong evidence that posttranslational gamma-carboxylation of VKD clotting factors was achieved in engineered Aspergillus strains. Further process optimization can lead to higher intracellular or extracellular gamma-carboxylated VKD protein yields.

### References:

Abas, L. and Luschnig, C. (2010). Anal Biochem, 401(2):217-227.
Bandyopadhyay, P. K. (2008). Vitam Horm, 78:157-184.
Bayram et al. (2008). Science, 320(5882):1504-1506.
Berger et al. (2006). Mol Microbiol, 59(2):433-446.
Berkner, K. L. (2008). Vitam Horm, 78:131-156.
Berkner et al. (2004). J Thromb Haemost, 2(12):2118-2132.
Broekhuijsen et al. (1993). J Biotechnol, 31(2):135-145.
Brown et al. (2000). J Biol Chem, 275(26):19795-19802.
Choi et al. (2003). Proc Natl Acad Sci U S A, 100(9):5022-5027.
Felenbok, B. (1991). J Biotechnol, 17(1):11-17.
Fleissner et al. (2010). Appl Microbiol Biotechnol, 87(4):1255-1270.
Furt et al. (2010). Plant J, 64(1):38-46
Gerngross, T. U. (2004). Nat Biotechnol, 22(11):1409-1414.
Gómez et al. (2003). Mol Microbiol, 50(1):277-289.
Goodstadt et al. (2004). Trends Biochem Sci, 29(6):289-292.
Gouka et al. (1997). Appl Microbiol Biotechnol, 47(1) :1-11.
Grillberger et al. (2009). Biotechnol J, 4(2) :186-201.
Gwynne et al. (1989). Biochem Soc Trans, 17(2):338-340.
Hallgren et al. (2002). Biochemistry, 41(50):15045-15055.
Hallgren et al. (2012). J Proteome Res.
Hamilton et al. (2006). Science, 313(5792):1441-1443.
Hansson et al. (2005). J Thromb Haemost, 3(12):2633-2648.
Hill et al. Fungal Genetics Newsletter 48 2001: 20-21
Jones et al. (1995). Arch Microbiol, 163(3):176-181.
Kainz, E. (2006). Steps towards humanizing fungal glycosylation. PhD thesis, Universitaet fuer Bodenkultur Wien.
Kainz et al. (2008). Appl Environ Microbiol, 74(4):1076-1086.
Kelleher et al. (1999). Anal Chem, 71(19):4250-4253.
Kinghorn et al. (1982). Mol Gen Genet, 186(1) :145-152.
Kinghorn and Unkles (1993). Aspergillus. Biotechnology handbooks, volume 7. Plenum Press New York and London.
Koda et al. (2005). J Biosci Bioeng, 100(5):531-537.
Laemmli, U. K. (1970). Nature, 227(5259):680-685.
Li et al. (2010). Nature, 463(7280):507-512.
Lubertozzi and Keasling (2009). Biotechnol Adv, 27(1) :53-75.
MacKenzie et al. (2004). Filamentous fungi as expression systems for heterologous proteins. In U. Kück (Ed.), The Mycota II (pp. 289-315). Springer.
Meyer et al. (2011). Biotechnol Lett, 33(3):469-476.
Neuhoff et al. (1985). Electrophoresis, 9(9):427-448.
Nikolaev et al. (2002). Fungal Genet Biol, 37(1) :89-97.
Oldenburg et al. (2006). Antioxid Redox Signal, 8(3-4):347-353.
Olsen, L. (2006). Monographie. Master's thesis, Universitaet fuer Bodenkultur Wien.
Pachlinger et al. (2005). Appl Environ Microbiol, 71(2):672-678.
Perkins et al. (1999). Electrophoresis, 20(18):3551-3567.
Pfaffl, M. W. (2001). Nucleic Acids Res, 29(9):e45.
Pipe, S. W. (2008). Thromb Haemost, 99(5):840-850.
Pipe, S. W. (2010). Hematology Am Soc Hematol Educ Program, 2010:203-209.
PONTECORVO et al. (1953). Adv Genet, 5:141-238.
Prenafeta-Boldu et al. (2006). FEMS Microbiol Rev, 30(1) :109-130.
Puig et al. (2001). Methods, 24(3):218-229.
Rasband, W. S. (1997-2011.). Imagej http://imagej.nih.gov/ij/.
Technical report, National Institutes of Health, Bethesda, Maryland, USA.
Reyes-Dominguez et al. (2008). Eukaryot Cell, 7(4):656-663.
Rishavy et al. (2011). J Biol Chem, 286(9):7267-7278.
Rost et al. (2004). Nature, 427(6974):537-541.
Sato et al. (2003). Mol Biol Cell, 14(9):3605-3616.
Shevchenko et al. (1996). Anal Chem, 68(5):850-858.
Sun et al. (2012). J Chromatogr B Analyt Technol Biomed Life Sci, 898:78-89.
Suttie, J. W. (2009). Vitamin K in health and disease. CRC Press.
Teasdale et al. (1996). Annu Rev Cell Dev Biol, 12:27-54.
Tie et al. (2000). Blood, 96 (3) : 973-978.
Tie et al. (2005). J Biol Chem, 280(16):16410-16416.
Jian-Ke Tie et al. (2011) Blood. 117(10): 2967-2974.
Tilburn et al. (1983). Gene, 26(2-3):205-221.
Tishler et al. (1942). JACS, 62:2866-2871.
Toews et al. (2004). Curr Genet, 45(6):383-389.
Towbin et al. (1979). Proc Natl Acad Sci U S A, 76(9):4350-4354.
Vatandoost et al. (2012). Biotechnol Prog, 28(1) :45-51.
Wajih et al. (2005). J Biol Chem, 280(36):31603-31607.
Wallin et al. (1982) J Biol Chem 257(4) :1583-1586.
Wallin et al. (1985). J Clin Invest, 76(5):1879-1884.
Wang et al. (2005). Mol Cancer Res, 3(6) :317-323.
Ward et al. (2006). Adv Appl Microbiol, 58:1-75.
Watanabe et al. (2007). Biosci Biotechnol Biochem, 71(11):2688-2696.
Westhofen et al. (2011). J Biol Chem, 286(17):15085-15094.

## Claims

1. A fungal cell transformed with exogenous nucleotide coding sequence of a gamma-carboxylase, a vitamin K reductase, and a vitamin K-dependent polypeptide, **characterized in that** the gamma-carboxylase and vitamin K reductase sequences lack an ER retention leader sequence endogenous to said fungus cell, preferably are free of any fungal ER retention leader sequence.

2. Fungal cell according to claim 1, **characterized in that** the coding sequence of the gamma-carboxylase comprises a sequence selected from SEQ ID NO: 1 or a sequence being at least 70% identical with the entire SEQ ID NO: 1.

3. Fungal cell according to claim 1 or 2, **characterized in that** the coding sequence of the vitamin K reductase comprises a sequence selected from SEQ ID NO: 4 or a sequence being at least 70% identical with the entire SEQ ID NO: 4, and/or a sequence with a V29L mutation or V29M mutation of the valine corresponding to V29 in a vitamin K reductase encoded by SEQ ID NO: 4.

4. Fungal cell comprising a gamma-carboxylase with a sequence as set forth in SEQ ID NO: 1 or a sequence being at least 70% identical with the entire SEQ ID NO: 1 and a vitamin K reductase with a sequence as set forth in SEQ ID NO: 4 or a sequence being at least 70% identical with the entire SEQ ID NO: 4, preferably further comprising a sequence of a vitamin K-dependent polypeptide.

5. Fungal cell according to claim to any one of claims 1 to 4, characterized as an *Aspergillus* cell, preferably an *Aspergillus nidulans* cell, a *A. oryzae* or *A*. *niger* cell, or a hyphal fungal cell, such as of *Trichoderma* spp., *Fusarium* spp. or *Neurospora* spp. or a yeast-type fungal cell, such as a *Saccharomyces, Schizosaccharomyces* or *Pichia* cell, in particular a *Pichia pastoris, Saccharomyces cerevisiae,* or *Schizosaccharomyces pombe* cell.

6. Fungal cell according to any one of claims 1 to 5, **characterized in that** the vitamin K-dependent polypeptide is selected from prothrombin, factor VII, factor IX, factor X, protein C, protein S, protein Z, pulmonary surfactant-associated proteins, osteocalcin, proline-rich GIa protein 1, and matrix gla-protein.

7. Fungal cell according to claim 6, **characterized in that** the vitamin K-dependent polypeptide is prothrombin and the prothrombin coding sequence comprises a sequence selected from SEQ ID NO: 7 or a sequence being at least 70% identical with the entire SEQ ID NO: 7.

8. Fungal cell according to any one of claims 1 to 7, **characterized in that** the gamma-carboxylase and the vitamin K reductase are expressed in a ratio of gamma-carboxylase to vitamin K reductase expression in the range of 1:20 to 20:1.

9. Fungal cell according to any one of claims 1 to 8, **characterized in that**, the genes of said gamma-carboxylase and vitamin K reductase comprise different promoters, preferably different inducible promoters which allow to set defined expression level ratios.

10. Fungal cell according to any one of claims 1 to 9 **characterized in that** said gamma-carboxylase and vitamin K reductase have been introduced into the genome of said cell in an amount ratio of gamma-carboxylase to vitamin K reductase integrations of 1:5 to 5:1.

11. A method of gamma-carboxylating a vitamin K-dependent polypeptide in a cell according to any one of claims 1 to 10, comprising expressing the gamma-carboxylase, vitamin K reductase, and vitamin K-dependent polypeptide in the presence of vitamin K or a vitamin K precursor, such as vitamin K hydroquinone, in said cell, preferably the method comprising culturing the cells in the presence of carbonate and/or phosphate.

12. Method of claim 11 comprising harvesting the carboxylated vitamin K-dependent polypeptide from the culture supernatant or from intracellular contents.

13. Method of claim 11 or 12 comprising administering vitamin K or said precursor to said cell, preferably wherein said vitamin K or precursor is provided in a micelle.

14. The method of any one of claims 11 to 13 wherein said cell expresses polypeptides according to SEQ ID NO: 3 (gamma-carboxylase) and SEQ ID NO: 6 (vitamin K reductase), preferably further a polypeptide of SEQ ID NO: 9 (prothrombin).

15. Method of manufacturing a cell according to any one of claims 1 to 10, comprising the step of providing a fungus cell and introducing into said cell an exogenous nucleotide coding sequence of a gamma-carboxylase and an exogenous nucleotide coding sequence a vitamin K reductase, wherein the gamma-carboxylase and vitamin K reductase sequences lack an ER retention sequence endogenous to said fungus cell, preferably are free of any fungal ER retention sequence, preferably the method comprising culturing the cells in the presence of carbonate and/or phosphate.


SEQUENCE LISTING

<110>  Baxter International Inc.
       Baxter Healthcare SA

<120>  Transformed fungus with vitamin K dependent activity

<130>  R 62292

<160>  9

<170>  PatentIn version 3.3

<210>  1
<211>  2274
<212>  DNA
<213>  Artificial

<220>
<223>  codon optimized GGCX coding seqeunce

<400>  1
atggccgtct ccgccggctc cgcccgcacc tccccttcct ccgacaaggt ccagaaggac      60

aaggccgagc tcatctccgg ccctcgccag gactcccgca tcggcaagct cctcggcttc     120

gagtggaccg acctctcctc ctggcgccgc ctcgtcaccc tcctcaaccg ccctaccgac     180

cctgcctccc tcgccgtctt ccgcttcctc ttcggcttcc tcatggtcct cgacatccct     240

caggagcgcg gcctctcctc cctcgaccgc aagtacctcg acggcctcga cgtctgccgc     300

ttccctctcc tcgacgccct ccgccctctc cctctcgact ggatgtacct cgtctacacc     360

atcatgttcc tcggcgccct cggcatgatg ctcggcctct gctaccgcat ctcctgcgtc     420

ctcttcctcc tcccttactg gtacgtcttc ctcctcgaca agacctcctg gaacaaccac     480

tcctacctct acggcctcct cgccttccag ctcaccttca tggacgccaa ccactactgg     540

tccgtcgacg gcctcctcaa cgcccaccgc cgcaacgccc acgtccctct ctggaactac     600

gccgtcctcc gcggccagat cttcatcgtc tacttcatcg ccggcgtcaa gaagctcgac     660

gccgactggg tcgagggcta ctccatggag tacctctccc gccactggct cttctcccct     720

ttcaagctcc tcctctccga ggagctcacc tccctcctcg tcgtccactg gggcggcctc     780

ctcctcgacc tctccgccgg cttcctcctc ttcttcgacg tctcccgctc catcggcctc     840

ttcttcgtct cctacttcca ctgcatgaac tcccagctct tctccatcgg catgttctcc     900

tacgtcatgc tcgcctcctc ccctctcttc tgctcccctg agtggcctcg caagctcgtc     960

tcctactgcc ctcgccgcct ccagcagctc ctccctctca aggccgcccc tcagccttcc    1020

gtctcctgcg tctacaagcg ctcccgcggc aagtccggcc agaagcctgg cctccgccac    1080

cagctcggcg ccgccttcac cctcctctac ctcctcgagc agctcttcct cccttactcc    1140

cacttcctca cccagggcta caacaactgg accaacggcc tctacggcta ctcctgggac    1200

atgatggtcc actcccgctc ccaccagcac gtcaagatca cctaccgcga cggccgcacc    1260

ggcgagctcg gctacctcaa ccctggcgtc ttcacccagt cccgccgctg gaaggaccac 1320

gccgacatgc tcaagcagta cgccacctgc ctctcccgcc tcctccctaa gtacaacgtc 1380

accgagcctc agatctactt cgacatctgg gtctccatca acgaccgctt ccagcagcgc 1440

atcttcgacc ctcgcgtcga catcgtccag gccgcctggt cccctttcca gcgcacctcc 1500

tgggtccagc ctctcctcat ggacctctcc ccttggcgcg ccaagctcca ggagatcaag 1560

tcctccctcg acaaccacac cgaggtcgtc ttcatcgccg acttccctgg cctccacctc 1620

gagaacttcg tctccgagga cctcggcaac acctccatcc agctcctcca gggcgaggtc 1680

accgtcgagc tcgtcgccga gcagaagaac cagaccctcc gcgagggcga gaagatgcag 1740

ctccctgccg gcgagtacca caaggtctac accacctccc cttccccttc ctgctacatg 1800

tacgtctacg tcaacaccac cgagctcgcc ctcgagcagg acctcgccta cctccaggag 1860

ctcaaggaga aggtcgagaa cggctccgag accggccctc tccctcctga gctccagcct 1920

ctcctcgagg gcgaggtcaa gggcggccct gagcctaccc ctctcgtcca gaccttcctc 1980

cgccgccagc agcgcctcca ggagatcgag cgccgccgca acaccccttt ccacgagcgc 2040

ttcttccgct tcctcctccg caagctctac gtcttccgcc gctccttcct catgacctgc 2100

atctccctcc gcaacctcat cctcggccgc ccttccctcg agcagctcgc ccaggaggtc 2160

acctacgcca acctccgccc tttcgaggcc gtcggcgagc tcaacccttc caacaccgac 2220

tcctcccact ccaaccctcc tgagtccaac cctgaccctg tccactccga gttc 2274

<210> 2
<211> 2274
<212> DNA
<213> Homo sapiens

<400> 2
atggcggtgt ctgccgggtc cgcgcggacc tcgcccagct cagataaagt acagaaagac 60

aaggctgaac tgatctcagg gcccaggcag gacagccgaa tagggaaact cttgggtttt 120

gagtggacag atttgtccag ttggcggagg ctggtgaccc tgctgaatcg accaacggac 180

cctgcaagct tagctgtctt tcgttttctt tttgggttct tgatggtgct agacattccc 240

caggagcggg ggctcagctc tctggaccgg aaataccttg atgggctgga tgtgtgccgc 300

ttccccttgc tggatgccct acgcccactg ccacttgact ggatgtatct tgtctacacc 360

atcatgtttc tgggggcact gggcatgatg ctgggcctgt gctaccggat aagctgtgtg 420

ttattcctgc tgccatactg gtatgtgttt ctcctggaca agacatcatg gaacaaccac 480

tcctatctgt atgggttgtt ggcctttcag ctaacattca tggatgcaaa ccactactgg 540

tctgtggacg gtctgctgaa tgcccatagg aggaatgccc acgtgcccct ttggaactat 600

gcagtgctcc gtggccagat cttcattgtg tacttcattg cgggtgtgaa aaagctggat 660

gcagactggg ttgaaggcta ttccatggaa tatttgtccc ggcactggct cttcagtccc 720

ttcaaactgc tgttgtctga ggagctgact agcctgctgg tcgtgcactg gggtgggctg 780

ctgcttgacc tctcagctgg tttcctgctc ttttttgatg tctcaagatc cattggcctg 840

ttctttgtgt cctacttcca ctgcatgaat tcccagcttt tcagcattgg tatgttctcc 900

tacgtcatgc tggccagcag ccctctcttc tgctcccctg agtggcctcg gaagctggtg 960

tcctactgcc cccgaaggtt gcaacaactg ttgcccctca aggcagcccc tcagcccagt 1020

gtttcctgtg tgtataagag gagccggggc aaaagtggcc agaagccagg gctgcgccat 1080

cagctgggag ctgccttcac cctgctctac ctcctggagc agctattcct gccctattct 1140

cattttctca cccagggcta taacaactgg acaaatgggc tgtatggcta ttcctgggac 1200

atgatggtgc actcccgctc ccaccagcac gtgaagatca cctaccgtga tggccgcact 1260

ggcgaactgg gctaccttaa ccctggggta tttacacaga gtcggcgatg gaaggatcat 1320

gcagacatgc tgaagcaata tgccacttgc ctgagccgcc tgcttcccaa gtataatgtc 1380

actgagcccc agatctactt tgatatttgg gtctccatca atgaccgctt ccagcagagg 1440

atttttgacc ctcgtgtgga catcgtgcag gccgcttggt caccctttca gcgcacatcc 1500

tgggtgcaac cactcttgat ggacctgtct ccctggaggg ccaagttaca ggaaatcaag 1560

agcagcctag acaaccacac tgaggtggtc ttcattgcag atttccctgg actgcacttg 1620

gagaattttg tgagtgaaga cctgggcaac actagcatcc agctgctgca gggggaagtg 1680

actgtggagc ttgtggcaga acagaagaac cagactcttc gagagggaga aaaaatgcag 1740

ttgcctgctg gtgagtacca taaggtgtat acgacatcac ctagcccttc ttgctacatg 1800

tacgtctatg tcaacactac agagcttgca ctggagcaag acctggcata tctgcaagaa 1860

ttaaaggaaa aggtggagaa tggaagtgaa acagggcctc tacccccaga gctgcagcct 1920

ctgttggaag gggaagtaaa agggggccct gagccaacac ctctggttca gacctttctt 1980

agacgccaac aaaggctcca ggagattgaa cgccggcgaa atactccttt ccatgagcga 2040

ttcttccgct tcttgttgcg aaagctctat gtctttcgcc gcagcttcct gatgacttgt 2100

atctcacttc gaaatctgat attaggccgt ccttccctgg agcagctggc ccaggaggtg 2160

acttatgcaa acttgagacc ctttgaggca gttggagaac tgaatccctc aaacacggat 2220

tcttcacatt ctaatcctcc tgagtcaaat cctgatcctg tccactcaga gttc 2274

<210> 3
<211> 758
<212> PRT
<213> Homo sapiens

<400> 3

<210>  4
<211>  489
<212>  DNA
<213>  Artificial

<220>
<223>  codon optimized VKORC1 coding sequence

<400>  4
atgggctcca cctggggctc ccctggctgg gtccgcctcg ccctctgcct caccggcctc      60

gtcctctccc tctacgccct ccatgtgaag gccgcccgcg cccgcgaccg cgactaccgc     120

gccctctgcg acgtcggcac cgccatctcc tgctcccgcg tcttctcctc ccgctggggc     180

cgcggcttcg gcctcgtcga gcacgtcctc ggccaggact ccatcctcaa ccagtccaac     240

tccatcttcg gctgcatctt ctacaccctc cagctcctcc tcggctgcct ccgcacccgc     300

tgggcctccg tcctcatgct cctctcctcc ctcgtctccc tcgccggctc cgtctacctc     360

gcctggattc tcttcttcgt cctctacgac ttctgcatcg tctgcatcac cacctacgcc     420

atcaacgtct ccctcatgtg gctctccttc cgcaaggtcc aggagcctca gggcaaggcc     480

aagcgccac                                                             489


<210>  5
<211>  489
<212>  DNA
<213>  Homo sapiens

<400>  5
atgggcagca cctgggggag ccctggctgg gtgcggctcg ctctttgcct gacgggctta      60

gtgctctcgc tctacgcgct gcacgtgaag gcggcgcgcg cccgggaccg ggattaccgc     120

gcgctctgcg acgtgggcac cgccatcagc tgttcgcgcg tcttctcctc caggtggggc     180

aggggtttcg ggctggtgga gcatgtgctg ggacaggaca gcatcctcaa tcaatccaac     240

agcatattcg gttgcatctt ctacacacta cagctattgt taggttgcct gcggacacgc     300

tgggcctctg tcctgatgct gctgagctcc ctggtgtctc tcgctggttc tgtctacctg     360

gcctggatcc tgttcttcgt gctctatgat ttctgcattg tttgtatcac cacctatgct     420

atcaacgtga gcctgatgtg gctcagtttc cggaaggtcc aagaacccca gggcaaggct     480

aagaggcac                                                             489


<210>  6
<211>  163
<212>  PRT
<213>  Homo sapiens

<400>  6

<210>  7
<211>  1866
<212>  DNA
<213>  Artificial

<220>
<223>  prothrombin codon optimized coding sequence

<400>  7
atggctcacg tccgcggcct ccagctccct ggctgcctcg ccctcgctgc tctctgctcc      60

ctcgtccact cccagcacgt cttcctcgcc cctcagcagg ctcgctccct cctccagcgc     120

gtccgccgcg ctaacacctt cctcgaggag gtccgcaagg gcaacctcga gcgcgagtgc     180

gtcgaggaga cctgctccta cgaggaggct ttcgaggccc tcgagtcctc caccgccacc     240

gacgtcttct gggctaagta caccgcctgc gagaccgctc gcacccctcg cgacaagctc     300

gctgcctgcc tcgagggcaa ctgcgctgag ggcctcggca ccaactaccg cggccacgtc 360

aacatcaccc gctccggcat cgagtgccag ctctggcgct cccgctaccc tcacaagcct 420

gagatcaact ccaccaccca ccctggcgcc gacctccagg agaacttctg ccgcaaccct 480

gactcctcca ccaccggccc ttggtgctac accaccgacc ctaccgtccg ccgccaggag 540

tgctccatcc ctgtctgcgg ccaggaccag gtcaccgtcg ccatgacccc tcgctccgag 600

ggctcctccg tcaacctctc ccctcctctc gagcagtgcg tccctgaccg cggccagcag 660

taccagggcc gcctcgccgt caccacccac ggcctccctt gcctcgcttg ggcctccgct 720

caggccaagg ctctctccaa gcaccaggac ttcaactccg ccgtccagct cgtcgagaac 780

ttctgccgca accctgacgg cgacgaggag ggcgtctggt gctacgtcgc cggcaagcct 840

ggcgacttcg gctactgcga cctcaactac tgcgaggagg ctgtcgagga ggagaccggc 900

gacggcctcg acgaggactc cgaccgcgcc atcgagggcc gcaccgctac ctccgagtac 960

cagaccttct tcaaccctcg caccttcggc tccggcgagg ctgactgcgg cctccgccct 1020

ctcttcgaga agaagtccct cgaggacaag accgagcgcg agctcctcga gtcctacatc 1080

gacggccgca tcgtcgaggg ctccgacgcc gagatcggca tgtccccttg gcaggtcatg 1140

ctcttccgca agtcccctca ggagctcctc tgcggcgctt ccctcatctc cgaccgctgg 1200

gtcctcaccg ctgcccactg cctcctctac cctccttggg acaagaactt caccgagaac 1260

gacctcctcg tccgcatcgg caagcactcc cgcacccgct acgagcgcaa catcgagaag 1320

atctccatgc tcgagaagat ctacatccac cctcgctaca actggcgcga gaacctcgac 1380

cgcgacatcg ccctcatgaa gctcaagaag cctgtcgcct tctccgacta catccaccct 1440

gtctgcctcc ctgaccgcga gaccgctgcc tccctcctcc aggccggcta caagggccgc 1500

gtcaccggct ggggcaacct caaggagacc tggaccgcta acgtcggcaa gggccagcct 1560

tccgtcctcc aggtcgtcaa cctccctatc gtcgagcgcc ctgtctgcaa ggactccacc 1620

cgcatccgca tcaccgacaa catgttctgc gctggctaca agcctgacga gggcaagcgc 1680

ggcgacgctt gcgagggcga ctccggcggc cctttcgtca tgaagtcccc tttcaacaac 1740

cgctggtacc agatgggcat cgtctcctgg ggcgagggct gcgaccgcga cggcaagtac 1800

ggcttctaca cccacgtctt ccgcctcaag aagtggatcc agaaggtcat cgaccagttc 1860

ggcgag 1866

<210> 8
<211> 1866
<212> DNA
<213> Homo sapiens

<400> 8
atggcgcacg tccgaggctt gcagctgcct ggctgcctgg ccctggctgc cctgtgtagc 60

cttgtgcaca gccagcatgt gttcctggct cctcagcaag cacggtcgct gctccagcgg 120

gtccggcgag ccaacacctt cttggaggag gtgcgcaagg gcaacctgga gcgagagtgc 180

gtggaggaga cgtgcagcta cgaggaggcc ttcgaggctc tggagtcctc cacggctacg 240

gatgtgttct gggccaagta cacagcttgt gagacagcga ggacgcctcg agataagctt 300

gctgcatgtc tggaaggtaa ctgtgctgag ggtctgggta cgaactaccg agggcatgtg 360

aacatcaccc ggtcaggcat tgagtgccag ctatggagga gtcgctaccc acataagcct 420

gaaatcaact ccactaccca tcctggggcc gacctacagg agaatttctg ccgcaacccc 480

gacagcagca ccacgggacc ctggtgctac actacagacc ccaccgtgag gaggcaggaa 540

tgcagcatcc ctgtctgtgg ccaggatcaa gtcactgtag cgatgactcc acgctccgaa 600

ggctccagtg tgaatctgtc acctccattg gagcagtgtg tccctgatcg ggggcagcag 660

taccaggggc gcctggcggt gaccacacat gggctcccct gcctggcctg ggccagcgca 720

caggccaagg ccctgagcaa gcaccaggac ttcaactcag ctgtgcagct ggtggagaac 780

ttctgccgca acccagacgg ggatgaggag ggcgtgtggt gctatgtggc cgggaagcct 840

ggcgactttg ggtactgcga cctcaactat tgtgaggagg ccgtggagga ggagacagga 900

gatgggctgg atgaggactc agacagggcc atcgaagggc gtaccgccac cagtgagtac 960

cagactttct tcaatccgag gacctttggc tcgggagagg cagactgtgg gctgcgacct 1020

ctgttcgaga agaagtcgct ggaggacaaa accgaaagag agctcctgga atcctacatc 1080

gacgggcgca ttgtggaggg ctcggatgca gagatcggca tgtcaccttg gcaggtgatg 1140

cttttccgga agagtcccca ggagctgctg tgtggggcca gcctcatcag tgaccgctgg 1200

gtcctcaccg ccgcccactg cctcctgtac ccgccctggg acaagaactt caccgagaat 1260

gaccttctgg tgcgcattgg caagcactcc cgcaccaggt acgagcgaaa cattgaaaag 1320

atatccatgt tggaaaagat ctacatccac cccaggtaca actggcggga gaacctggac 1380

cgggacattg ccctgatgaa gctgaagaag cctgttgcct tcagtgacta cattcaccct 1440

gtgtgtctgc ccgacaggga gacggcagcc agcttgctcc aggctggata caaggggcgg 1500

gtgacaggct ggggcaacct gaaggagacg tggacagcca acgttggtaa ggggcagccc 1560

agtgtcctgc aggtggtgaa cctgcccatt gtggagcggc cggtctgcaa ggactccacc 1620

cggatccgca tcactgacaa catgttctgt gctggttaca agcctgatga agggaaacga 1680

ggggatgcct gtgaaggtga cagtggggga ccctttgtca tgaagagccc ctttaacaac 1740

cgctggtatc aaatgggcat cgtctcatgg ggtgaaggct gtgaccggga tgggaaatat 1800

ggcttctaca cacatgtgtt ccgcctgaag aagtggatac agaaggtcat tgatcagttt 1860

ggagag 1866

<210>  9
<211>  622
<212>  PRT
<213>  Homo sapiens

<400>  9